# EUROPEAN PATENT APPLICATION

(11) **EP 4 751 729 A2**
(43) Date of publication of application: **03.06.2026**
(21) Application number: 26170071.0
(22) Date of filing: 05.09.2017
(51) Int. Cl.: A61K 38/00

(54) **ALLERGY ANTIGEN AND EPITOPE THEREOF**

(30) Priority: 03.03.2017 WO PCT/JP2017/008593
(62) Divisional of application: 17898509.9
(71) Applicant: Hoyu Co., Ltd., Nagoya-shi, Aichi 461-8650 (JP)
(72) Inventor: MATSUNAGA, Kayoko, Toyoake-shi, 470-1192 (JP); YAGAMI, Akiko, Toyoake-shi, 470-1192 (JP); SHIMOJO, Naoshi, Nagakute-shi, 480-1136 (JP); OHNO, Fumiaki, Nagakute-shi, 480-1136 (JP)
(74) Representative: Mewburn Ellis LLP

(57) **Abstract**

The present invention provides novel antigens of an allergy to soybean, methods and kits for diagnosing an allergy to soybean, pharmaceutical compositions comprising such an antigen, soybeans or processed products of soybean in which such an antigen is eliminated, and a tester for determining the presence or absence of a soybean antigen in an object of interest. The present invention also relates to polypeptides comprising an epitope of an allergen, kits, compositions and methods for diagnosing an allergy, comprising such a polypeptide, pharmaceutical compositions comprising such a polypeptide, and food raw materials or edible processed products in which an antigen comprising such a polypeptide is eliminated or reduced, or the polypeptide is cleaved or removed. The present invention further relates to a tester for determining the presence or absence of an antigen in an object of interest.

## Description

### TECHNICAL FIELD

The present invention relates to a novel antigen of an allergy to soybean. The present invention also relates to a kit, a composition, and a method for diagnosing allergy to soybean. The present invention also relates to a pharmaceutical composition comprising such an antigen and soybean or processed products of soybean in which such an antigen is eliminated or reduced. The present invention further relates to a tester for determining the presence or absence of a soybean antigen in an object of interest.

The present invention also relates to a polypeptide comprising an epitope of an allergen. The present invention also relates to a kit, a composition and a method for diagnosing an allergy, comprising such a polypeptide. The present invention also relates to a pharmaceutical composition comprising such a polypeptide, and a food raw material or an edible processed product in which an antigen comprising such a polypeptide is eliminated or reduced, or such a polypeptide is cleaved or removed. The present invention further relates to a method for producing an edible processed product in which such an antigen is eliminated or reduced. The present invention further relates to a tester for determining the presence or absence of an antigen comprising such a polypeptide in an object of interest.

### BACKGROUND ART

In blood and tissues of allergic patients, IgE antibodies specific to particular antigens are produced. Physiological consequences caused by interaction between such IgE antibodies and such particular antigens elicit allergic reactions.

In the process of production of conventional allergy testing agents, antigen reagents are commonly prepared simply by grinding a candidate allergenic food, material or the like (Patent Literature 1). As seen above, conventional antigen reagents do not necessarily contain only a particular antigenic protein inducing an allergic reaction (allergen component), and rather contain different types of protein components. Thus, conventional antigen reagents contain varied amounts of allergen components. For this reason, the only case where conventional allergy tests have permitted detection of a positive allergic reaction is when in a conventional antigen reagent containing many types of proteins, a particular protein acting as an allergen component is present in an amount exceeding a threshold that allows determination of a positive reaction for binding to an IgE antibody. However, no determination of a positive reaction was possible and diagnosis efficiency was not sufficiently high when using a conventional allergy testing agent in patients possessing an IgE antibody binding to an allergen component present in small amounts in an allergen such as food.

The severity and symptoms of an allergic reaction do not necessarily correlate with the content of an allergen component. Even when a patient's IgE antibody reacts with an allergen component present in trace amounts in a candidate allergic food and material, the allergic reaction may develop allergic symptoms or may affect the severity of those symptoms.

An attempt to increase the diagnostic efficiency is being made by examining IgE antibodies to components composing a crude antigen to distinguish sensitization that directly contributes to a diagnosis from sensitization based on cross-antigenicity by a pan-allergen or the like. 8 soybean allergens are currently registered to the World Health Organization/International Union of Immunological Societies (WHO/IUIS).

However, while it is necessary to exhaustively identify allergen components in candidate allergic foods and materials in order to enhance the reliability of allergy tests, the patient detection rate by the measurement of such allergenic components is far from sufficient. Identification of novel allergens in soybean is very important not only for increasing the precision of diagnosis, but also for determining targets of therapeutic agents and low allergenic foods.

Meanwhile, in the field of protein separation and purification, various efforts have conventionally been made to develop methods for separating and purifying a protein or nucleic acid of interest from cell extracts or the like. Such methods may well be exemplified by dialysis based on salt concentration, and centrifugal separation.

Other efforts have been made to develop many purification methods based on electric charges of protein or nucleic acid residues or on the difference in molecular weight. Electric charge-based purification methods can be exemplified by column chromatography using ion exchange resins, and isoelectric focusing. Purifications based on molecular weight difference can be exemplified by centrifugal separation, molecular-sieve column chromatography, and SDS-PAGE (sodium dodecyl sulfate-polyacrylamide gel electrophoresis).

In recent years, a method for separating and purifying many different proteins from a small amount of sample has been used, which is more specifically a two-dimensional electrophoresis consisting of isoelectric focusing in the first dimension, followed by SDS-PAGE in the second dimension. The present applicant has conventionally developed some 2D electrophoresis methods with high separation ability (Patent Literature 2-5).

Allergen-specific IgE antibodies recognize and bind to epitopes that are particular amino acid sequences in allergen components. However, only a slight number of analyses have been made on epitopes as to the allergen components (Non Patent Literature 1), but such analyses are still totally quite rare. Furthermore, any kit for diagnosing an allergy using a polypeptide comprising an epitope has not yet emerged in the market.

### CITATION LIST

### PATENT LITERATURE

PTL1: Japanese Patent Application Publication No. JP 2002-286716
PTL2: Japanese Patent Application Publication No. JP 2011-33544
PTL3: Japanese Patent Application Publication No. JP 2011-33546
PTL4: Japanese Patent Application Publication No. JP 2011-33547
PTL5: Japanese Patent Application Publication No. JP 2011-33548

### NON PATENT LITERATURE

NPL 1: Matsuo, H., et al., J. Biol. Chem., (2004), Vol.279, No.13, pp.12135-12140
NPL 2: Zhao, L., et al., PLoS One. 2017 Aug 11; 12(8):e0182935. doi: 10.1371/journal.pone.0182935. eCollection 2017.
NPL 3: Cong, Y., et al., J Dairy Sci. 2013; 96(11):6870-6. doi: 10.3168/jds.2013-6880. Epub 2013 Sep 12.
NPL 4: Beezhold, D. H., et al., J Allergy Clin Immunol. 2001 Jun; 107(6):1069-76.

### SUMMARY OF INVENTION

### TECHNICAL PROBLEM

The present invention provides novel antigens of an allergy to soybean. The present invention also provides methods and kits for diagnosing allergy to soybean. The present invention also provides pharmaceutical compositions comprising such an antigen and soybean or processed products of soybean in which such an antigen is eliminated or reduced. The present invention further provides testers for determining the presence or absence of a soybean antigen in an object of interest.

The present invention also provides polypeptides comprising an epitope of an allergen. The present invention also provides kits, compositions and methods for diagnosing an allergy. The present invention also provides pharmaceutical compositions comprising such a polypeptide, and food raw materials or edible processed products in which an antigen comprising such a polypeptide is eliminated or reduced, or such a polypeptide is cleaved or removed. The present invention further relates to methods for producing an edible processed product in which such an antigen is eliminated or reduced. The present invention further relates to testers for determining the presence or absence of an antigen comprising such a polypeptide in an object of interest.

### SOLUTION TO PROBLEM

In order to solve the aforementioned problems, the present inventors had made intensive studies to identify causative antigens of an allergy. As a result, the inventors succeeded in identifying novel antigens to which an IgE antibody in the serum of an allergic patient specifically binds. The present invention has been completed based on this finding.

Thus, in one embodiment, the present invention can be as defined below.
[1] A kit for diagnosing an allergy to a soybean, the kit comprising, as an antigen, at least one of proteins defined below in (35α) or (48α):
   (35α) (35αA1) elongation factor 1-alpha or a variant thereof, which is defined below in any of (35αA1-a) to (35αA1-e):
      (35αA1-a) a protein comprising an amino acid sequence with deletion, substitution, insertion or addition of one or several amino acids in SEQ ID NO: 460;
      (35αA1-b) a protein comprising an amino acid sequence having at least 70% identity to the amino acid sequence of SEQ ID NO: 460;
      (35αA1-c) a protein comprising an amino acid sequence encoded by a nucleotide sequence with deletion, substitution, insertion or addition of one or several nucleotides in SEQ ID NO: 459;
      (35αA1-d) a protein comprising an amino acid sequence encoded by a nucleotide sequence having at least 70% identity to the nucleotide sequence of SEQ ID NO: 459; or
      (35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
      (35αB1) a protein comprising at least one amino acid sequence selected from the group consisting of SEQ ID NOs: 461-466;
      (35αA2) elongation factor 1-alpha or a variant thereof, which is defined below in any of (35αA2-a) to (35αA2-e):
         (35αA2-a) a protein comprising an amino acid sequence with deletion, substitution, insertion or addition of one or several amino acids in SEQ ID NO: 468;
         (35αA2-b) a protein comprising an amino acid sequence having at least 70% identity to the amino acid sequence of SEQ ID NO: 468;
         (35αA2-c) a protein comprising an amino acid sequence encoded by a nucleotide sequence with deletion, substitution, insertion or addition of one or several nucleotides in SEQ ID NO: 467;
         (35αA2-d) a protein comprising an amino acid sequence encoded by a nucleotide sequence having at least 70% identity to the nucleotide sequence of SEQ ID NO: 467; or
         (35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
         (35αB2) a protein comprising at least one amino acid sequence selected from the group consisting of SEQ ID NOs: 469-471;
         (35αA3) elongation factor-1A isoform X1 or a variant thereof, which is defined below in any of (35αA3-a) to (35αA3-e):
            (35αA3-a) a protein comprising an amino acid sequence with deletion, substitution, insertion or addition of one or several amino acids in SEQ ID NO: 473;
            (35αA3-b) a protein comprising an amino acid sequence having at least 70% identity to the amino acid sequence of SEQ ID NO: 473;
            (35αA3-c) a protein comprising an amino acid sequence encoded by a nucleotide sequence with deletion, substitution, insertion or addition of one or several nucleotides in SEQ ID NO: 472;
            (35αA3-d) a protein comprising an amino acid sequence encoded by a nucleotide sequence having at least 70% identity to the nucleotide sequence of SEQ ID NO: 472; or
            (35αA3-e) a protein comprising an amino acid sequence encoded by a nucleic acid that hybridizes under stringent conditions with a nucleic acid having a nucleotide sequence complementary to the nucleotide sequence of SEQ ID NO: 472; or
            (35αB3) a protein comprising at least one amino acid sequence selected from the group consisting of SEQ ID NOs: 474-477;
   (48α) (48αA) uncharacterized protein LOC100306570 or a variant thereof, which is defined below in any of (48αA-a) to (48αA-e):
      (48αA-a) a protein comprising an amino acid sequence with deletion, substitution, insertion or addition of one or several amino acids in SEQ ID NO: 565;
      (48αA-b) a protein comprising an amino acid sequence having at least 70% identity to the amino acid sequence of SEQ ID NO: 565;
      (48αA-c) a protein comprising an amino acid sequence encoded by a nucleotide sequence with deletion, substitution, insertion or addition of one or several nucleotides in SEQ ID NO: 564;
      (48αA-d) a protein comprising an amino acid sequence encoded by a nucleotide sequence having at least 70% identity to the nucleotide sequence of SEQ ID NO: 564; or
      (48αA-e) a protein comprising an amino acid sequence encoded by a nucleic acid that hybridizes under stringent conditions with a nucleic acid having a nucleotide sequence complementary to the nucleotide sequence of SEQ ID NO: 564; or
      (48αB) a protein comprising at least one amino acid sequence selected from the group consisting of SEQ ID NOs: 566-570.
[2] A composition for diagnosing an allergy to a soybean, comprising, as an antigen, at least one of proteins as defined above in (35α) or (48α) of [1].
[3] A method for providing an indicator for diagnosing an allergy to a soybean in a subject, the method comprising the steps of:
   (i) contacting a sample obtained from the subject with an antigen, wherein the sample is a solution comprising an Ig antibody;
   (ii) detecting binding between the IgE antibody present in the sample from the subject and the antigen; and
   (iii) when the binding between the IgE antibody in the subject and the antigen is detected, an indicator of the fact that the subject is allergic to a soybean is provided;
   wherein the antigen is at least one of proteins as defined above in (35α) or (48α) of [1].
[4] A pharmaceutical composition comprising at least one of proteins as defined above in any of (35α) or (48α) of [1].
[5] The pharmaceutical composition as set forth in [4], wherein the pharmaceutical composition is intended for the treatment of an allergy to a soybean.
[6] A soybean or processed products of soybean in which an antigen is eliminated or reduced, wherein the antigen is at least one of proteins as defined above in any of (35α) or (48α) of [1].
[7] A method for producing processed products of soybean in which an antigen is eliminated or reduced, the method comprising the step of confirming that the antigen is eliminated or reduced in a production process of the processed products, wherein the antigen is at least one of proteins as defined above in (35α) or (48α) of [1].
[8] A tester for determining the presence or absence of a soybean antigen in an object of interest, comprising an antibody that binds to at least one of proteins as defined above in (35α) or (48α) of [1].
[9] A soybean-derived antigen which is at least one of proteins as defined above in (35α) or (48α) of [1] and is causative of an allergy to soybean.

The present inventors also succeeded in finding epitopes as to the novel antigens.

As mentioned above, interaction between IgE antibodies from allergic patients and particular antigens elicit allergic reactions. These IgE antibodies recognize and bind to epitopes that are particular amino acid sequences in allergen components. Since the epitopes have a relatively short amino acid sequence, the IgE antibodies are capable of binding to different allergen components if the same amino acid sequence is present in the different allergen components. When different allergen components have a common epitope so that IgE antibodies from allergic patients bind to both of them, the allergens have cross-reactivity (or also called cross-antigenicity). Thus, the epitopes defined in the present invention enable diagnosis or treatment of an allergy including cross-reactivity, and detection of a plurality of allergen components comprising the epitopes, etc.

The present invention has been completed based on this finding. Thus, in another embodiment, the present invention can be as defined below.
[10] A polypeptide specifically binding to an IgE antibody from an allergic patient, the polypeptide being (35β) or (48β) of the following:
   (35β) a polypeptide comprising at least one amino acid sequence selected from the group consisting of SEQ ID NOs: 847-856; and
   (48β) a polypeptide comprising at least one amino acid sequence selected from the group consisting of SEQ ID NOs: 919-922.
[11] A polypeptide specifically binding to an IgE antibody from an allergic patient, the polypeptide being any one of the following:
   (3β) a polypeptide comprising at least one amino acid sequence selected from the group consisting of SEQ ID NOs: 693-716;
   (20β) a polypeptide comprising at least one amino acid sequence selected from the group consisting of SEQ ID NOs: 788-792;
   (21β) a polypeptide comprising at least one amino acid sequence selected from the group consisting of SEQ ID NOs: 793-802;
   (22β) a polypeptide comprising at least one amino acid sequence selected from the group consisting of SEQ ID NOs: 803-808;
   (23β) a polypeptide comprising at least one amino acid sequence selected from the group consisting of SEQ ID NOs: 809-812;
   (24β) a polypeptide comprising the amino acid sequence of SEQ ID NO: 813;
   (44β) a polypeptide comprising at least one amino acid sequence selected from the group consisting of SEQ ID NOs: 907-909;
   (49β) a polypeptide comprising the amino acid sequence of SEQ ID NO: 923;
   (57β) a polypeptide comprising at least one amino acid sequence selected from the group consisting of SEQ ID NOs: 953-955;
   (58β) a polypeptide comprising at least one amino acid sequence selected from the group consisting of SEQ ID NOs: 956-960;
   (59β) a polypeptide comprising at least one amino acid sequence selected from the group consisting of SEQ ID NOs: 961 and 962; and
   (60β) a polypeptide comprising the amino acid sequence of SEQ ID NO: 963.
[12] The polypeptide according to the above [10] or [11], wherein the number of amino acid residues is 500 or less.
[13] A kit for diagnosing an allergy, comprising at least one of polypeptides according to any one of the above [10] to [12].
[14] A composition for diagnosing an allergy, the composition comprising at least one of polypeptides according to any one of the above [10] to [12] as an antigen.
[15] A method for providing an indicator for diagnosing an allergy in a subject, the method comprising the steps of:
   (i) contacting a sample obtained from the subject with an antigen, wherein the sample is a solution comprising an Ig antibody;
   (ii) detecting binding between the IgE antibody present in the sample obtained from the subject and the antigen; and
   (iii) when the binding between the IgE antibody in the subject and the antigen is detected, an indicator of the fact that the subject is allergic is provided;
   wherein the antigen is at least one of polypeptides according to any one of the above [10] to [12].
[16] A pharmaceutical composition comprising at least one of polypeptides according to any one of the above [10] to [12].
[17] The pharmaceutical composition according to the above [16], wherein the pharmaceutical composition is intended for the treatment of an allergy.
[18] A tester for determining the presence or absence of an antigen in an object of interest, the tester comprising an antibody that binds to at least one of polypeptides according to any one of the above [10] to [12].
[19] A food raw material or an edible processed product in which an antigen is eliminated or reduced, or a polypeptide is cleaved or removed, wherein the antigen is at least one of polypeptides according to any one of the above [10] to [12].
[20] A method for producing an edible processed product in which an antigen is eliminated or reduced, or a polypeptide is cleaved or removed, the method comprising the step of confirming that the antigen is eliminated or reduced, or the polypeptide is cleaved or removed, in a production process of the processed product, wherein the antigen is at least one of polypeptides according to any one of the above [10] to [12].

### ADVANTAGEOUS EFFECTS OF INVENTION

The present invention can provide novel antigens of an allergy to soybean. Since the novel antigens (allergen components) that trigger a soybean allergy were identified according to this invention, this invention can provide highly sensitive methods and kits for diagnosing an allergy to soybean, pharmaceutical compositions comprising such an antigen, soybean or processed products of soybean in which such an antigen is eliminated or reduced.

The present invention can provide novel polypeptides comprising an epitope of an allergen. Use of the polypeptide of the present invention enables provision of highly sensitive methods and kits for diagnosing an allergy, comprising such a polypeptide, pharmaceutical compositions comprising such a polypeptide, food raw materials or edible processed products in which an antigen comprising such a polypeptide is eliminated or reduced, or such a polypeptide is cleaved or removed, and a method for producing the edible processed products.

### BRIEF DESCRIPTION OF DRAWINGS

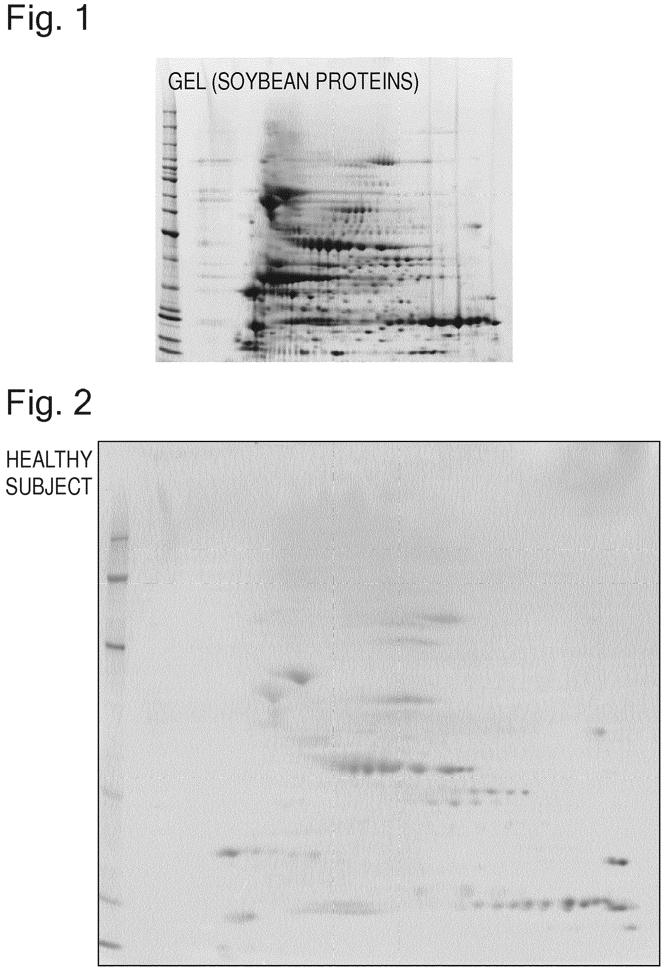

[Fig. 1] Fig. 1 is a photograph of a gel showing a protein electrophoretic pattern in two-dimensional electrophoresis of proteins contained in soybean. The bands at the left of the photograph are bands of molecular weight markers.
[Fig. 2] Fig. 2 is a photograph of an immunoblot of a two-dimensional electrophoretic pattern of proteins contained in soybean stained with serum of a healthy subject.

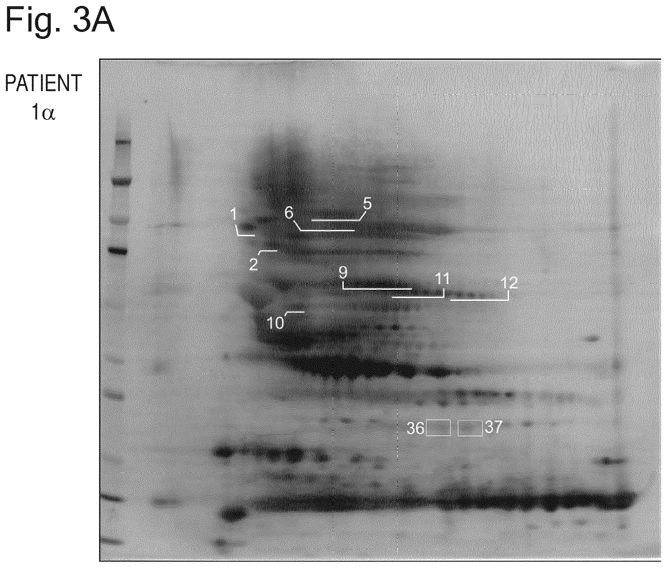

[Fig. 3A] Fig. 3A is a photograph of an immunoblot of a two-dimensional electrophoretic pattern of proteins contained in soybean stained with serum of Soybean-allergic patient 1α. The spots indicated with a white line or enclosed in a square are spot where the serum of the soybean-allergic patient specifically reacted. The numbers (n) indicated are the numbers of the spots and correspond to the spots indicated with "nα" herein.

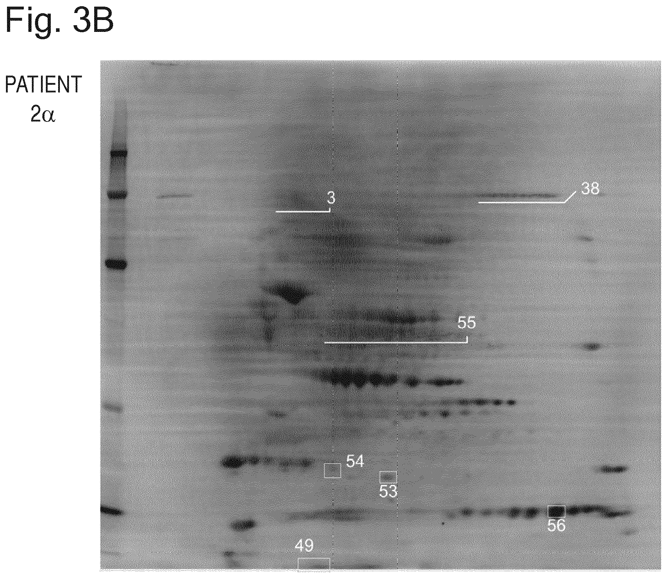

[Fig. 3B] Fig. 3B is a photograph of an immunoblot of a two-dimensional electrophoretic pattern of proteins contained in soybean stained with serum of Soybean-allergic patient 2α. The spots indicated with a white line or enclosed in a square are spot where the serum of the soybean-allergic patient specifically reacted. The numbers (n) indicated are the numbers of the spots and correspond to the spots indicated with "nα" herein.

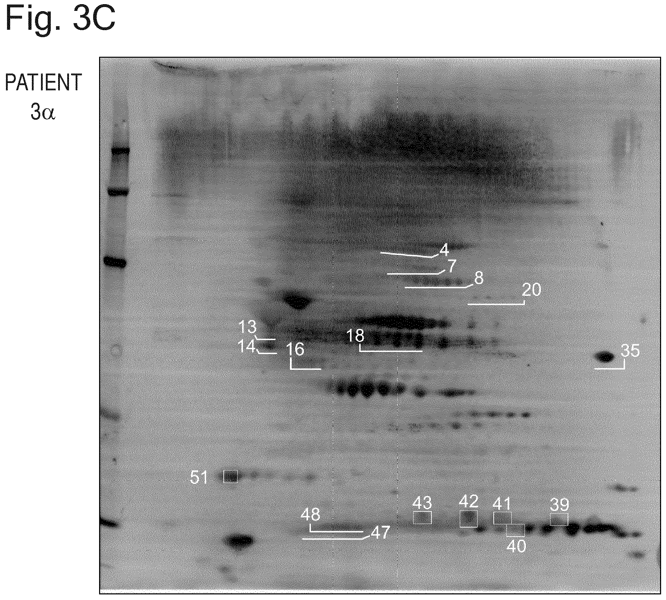

[Fig. 3C] Fig. 3C is a photograph of an immunoblot of a two-dimensional electrophoretic pattern of proteins contained in soybean stained with serum of Soybean-allergic patient 3α. The spots indicated with a white line or enclosed in a square are spot where the serum of the soybean-allergic patient specifically reacted. The numbers (n) indicated are the numbers of the spots and correspond to the spots indicated with "nα" herein.

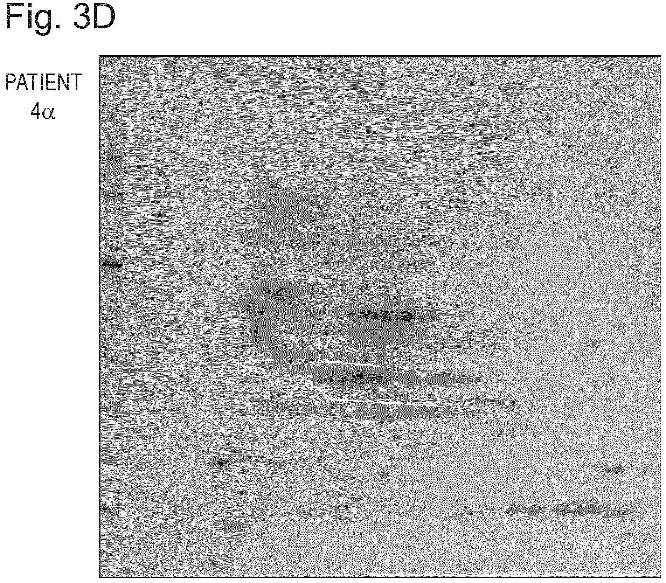

[Fig. 3D] Fig. 3D is a photograph of an immunoblot of a two-dimensional electrophoretic pattern of proteins contained in soybean stained with serum of Soybean-allergic patient 4α. The spots indicated with a while line are spots where the serum of the soybean-allergic patient specifically reacted. The numbers (n) indicated are the numbers of the spots and correspond to the spots indicated with "nα" herein.

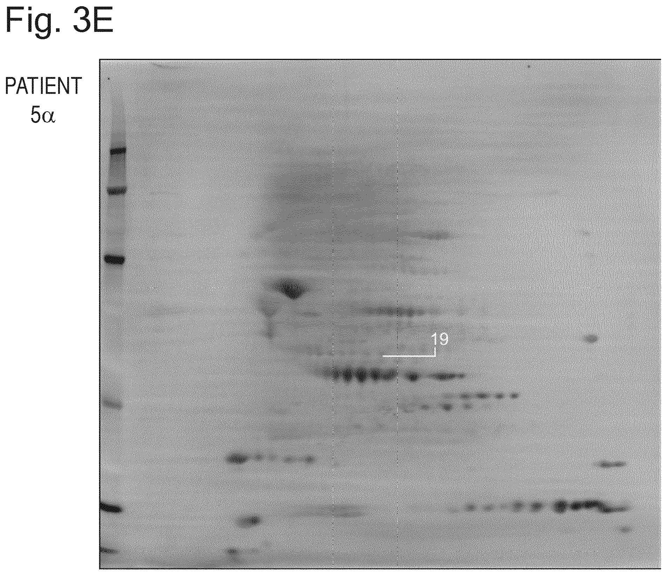

[Fig. 3E] Fig. 3E is a photograph of an immunoblot of a two-dimensional electrophoretic pattern of proteins contained in soybean stained with serum of Soybean-allergic patient 5α. The spots indicated with a while line are spots where the serum of the soybean-allergic patient specifically reacted. The numbers (n) indicated are the numbers of the spots and correspond to the spots indicated with "nα" herein.

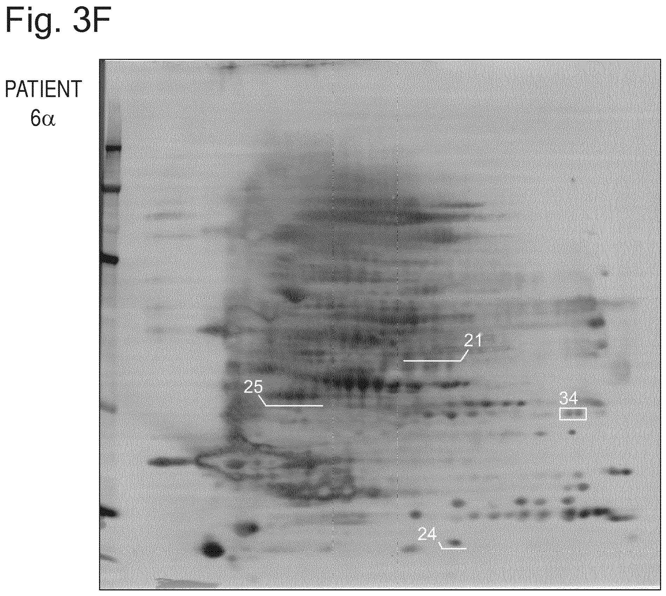

[Fig. 3F] Fig. 3F is a photograph of an immunoblot of a two-dimensional electrophoretic pattern of proteins contained in soybean stained with serum of Soybean-allergic patient 6α. The spots indicated with a white line or enclosed in a square are spot where the serum of the soybean-allergic patient specifically reacted. The numbers (n) indicated are the numbers of the spots and correspond to the spots indicated with "nα" herein.

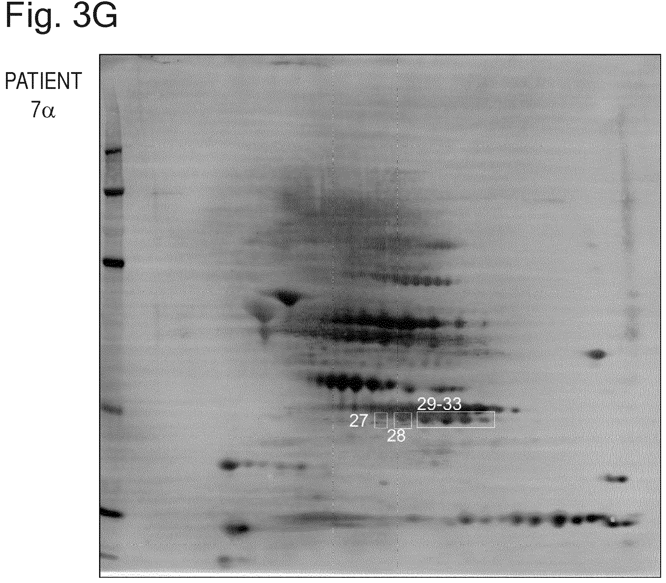

[Fig. 3G] Fig. 3G is a photograph of an immunoblot of a two-dimensional electrophoretic pattern of proteins contained in soybean stained with serum of Soybean-allergic patient 7α. The spots enclosed in a square are spots where the serum of the soybean-allergic patient specifically reacted. The numbers (n) indicated are the numbers of the spots and correspond to the spots indicated with "nα" herein.

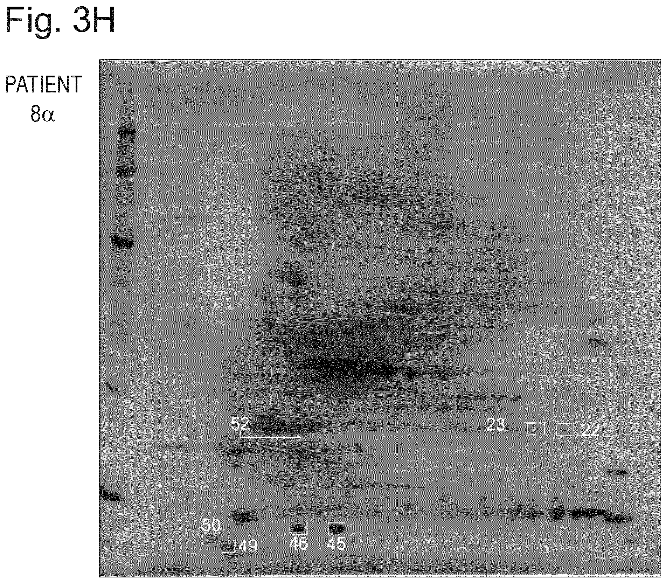

[Fig. 3H] Fig. 3H is a photograph of an immunoblot of a two-dimensional electrophoretic pattern of proteins contained in soybean stained with serum of Soybean-allergic patient 8α. The spots indicated with a white line or enclosed in a square are spot where the serum of the soybean-allergic patient specifically reacted. The numbers (n) indicated are the numbers of the spots and correspond to the spots indicated with "nα" herein.

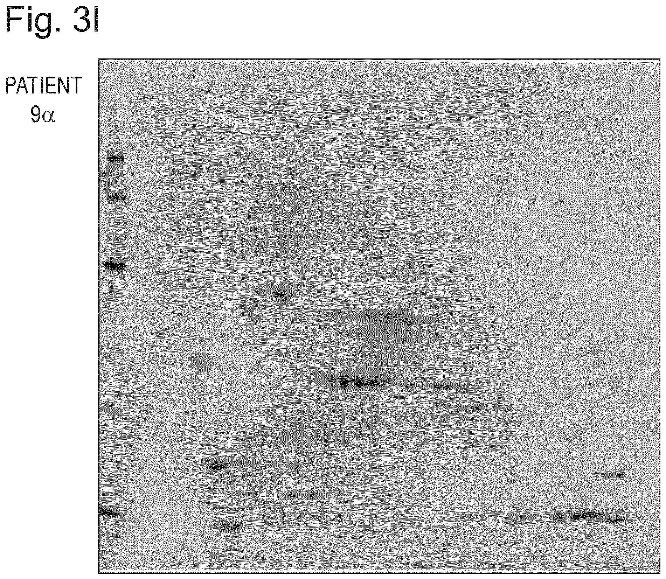

[Fig. 3I] Fig. 3I is a photograph of an immunoblot of a two-dimensional electrophoretic pattern of proteins contained in soybean stained with serum of Soybean-allergic patient 9α. The spots indicated with a white line or enclosed in a square are spot where the serum of the soybean-allergic patient specifically reacted. The numbers (n) indicated are the numbers of the spots and correspond to the spots indicated with "nα" herein.

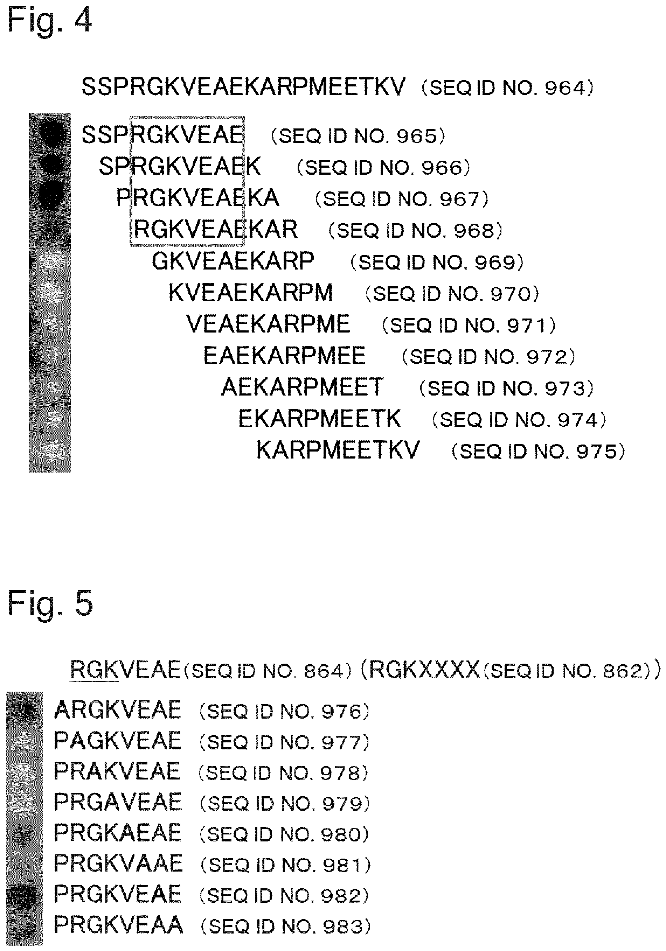

[Fig. 4] Fig. 4 is a diagram showing one example of results of determining peptides having the minimum number of amino acids functioning as an epitope by an overlapping technique.
[Fig. 5] Fig. 5 is a diagram showing one example of results of studying positions important for exertion of antigenicity as to the identified epitopes by an alanine scanning technique.

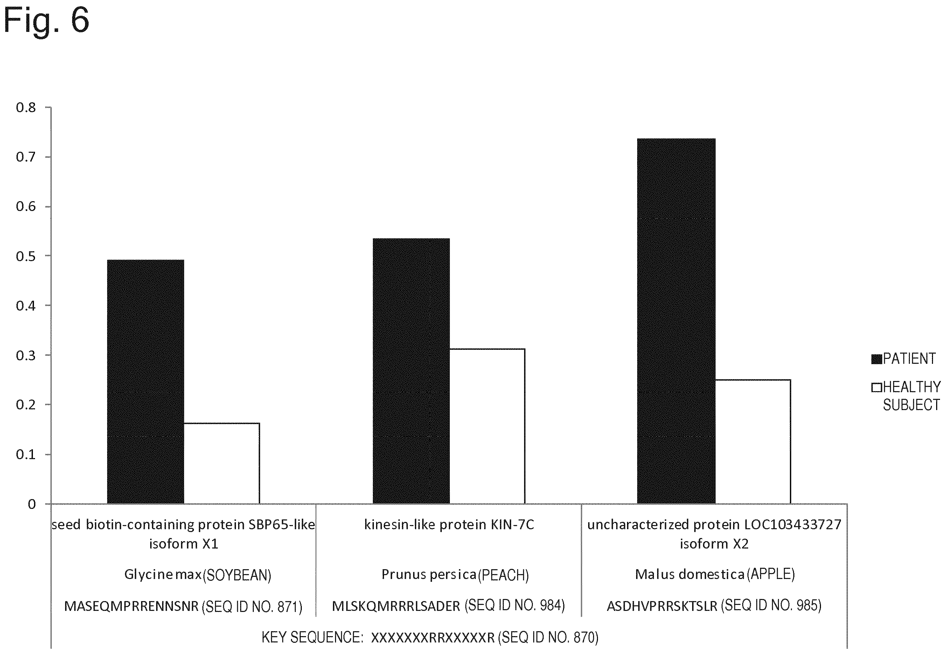

[Fig. 6] Fig. 6 is a graph showing results of studying cross-reactivity.

### DESCRIPTION OF EMBODIMENTS

The present invention will be described in detail below, but the present invention is not limited to them.

Unless otherwise defined herein, all scientific and technical terms used in relation to the present invention shall have meanings commonly understood by those skilled in the art.

Amino acid sequences herein are represented by one-letter amino acid symbols well known to those skilled in the art and the left end corresponds to the amino terminal and the right end corresponds to the carboxy terminal.

As referred to herein, the "allergy" refers to the state in which, when a certain antigen enters the body of a living individual sensitized to said antigen, the living individual shows a hypersensitive reaction detrimental to him/her. In blood and tissues of individuals with many food-allergic diseases, IgE antibodies specific to antigens are produced. IgE antibodies bind to mast cells or basophils. When an antigen specific to such an IgE antibody enters again the body of a patient with an allergic disease, said antigen combines with the IgE antibody bound to mast cells or basophils, and the IgE antibody crosslinks said antigen on the cell surface, resulting in physiological effects of IgE antibody-antigen interaction. Examples of such physiological effects include release of histamine, serotonin, heparin, eosinophil chemotactic factors, leucotrienes, or the like. These released substances provoke an allergic reaction resulting from the combination of an IgE antibody with particular antigens. Such allergic reactions caused by particular antigens occur through the aforementioned pathway.

In the present invention, the allergy of interest is not particularly limited as long as it is an allergy to an allergen comprising an epitope to be used. Such an allergy may include allergies to plants of the family *Malvaceae,* the family *Rubiaceae,* the family *Brassicaceae,* the family *Poaceae,* the family *Cucurbitaceae,* the family *Compositae,* the family *Asparagaceae,* the family *Juglandaceae,* the family *Rhamnaceae,* the family *Pedaliaceae,* the family *Umbelliferae,* the family *Euphorbiaceae,* the family *Solanaceae,* the family *Bromeliaceae,* the family *Caricaceae,* the family *Rosaceae,* the family *Amaranthaceae,* the family *Vitaceae,* the family *Leguminosae,* the family *Lythraceae* and/or the family *Betulaceae.* Examples of the plant of the family *Malvaceae* include nalta jute (Jew's mallow, scientific name: *Corchorus olitorius)* and cacao (scientific name: *Theobroma cacao).* Examples of the plant of the family *Rubiaceae* include Robusta coffee (scientific name: *Coffea canephora*). Examples of the plant of the family *Brassicaceae* include Qing geng cai (scientific name: *Brassica rapa),* radish (scientific name: *Raphanus sativus),* and rapeseed (scientific name: *Brassica napus).* Examples of the plant of the family *Poaceae* include great millet (scientific name: *Sorghum bicolor),* corn (scientific name: *Zea mays),* and bread wheat (scientific name: *Triticum aestivum).* Examples of the plant of the family *Cucurbitaceae* include melon (scientific name: *Cucumis melo),* bitter melon (bitter gourd, scientific name: *Momordica charantia),* and cucumber (scientific name: *Cucumis sativus).* Examples of the plant of the family *Compositae* include sunflower (scientific name: *Helianthus annuus).* Examples of the plant of the family *Asparagaceae* include asparagus (scientific name: *Asparagus officinalis).* Examples of the plant of the family *Juglandaceae* include Persian walnut (scientific name: *Juglans regia).* Examples of the plant of the family *Rhamnaceae* include Chinese date (scientific name: *Ziziphus jujuba*). Examples of the plant of the family *Pedaliaceae* include sesame (scientific name: *Sesamum indicum).* Examples of the plant of the family *Umbelliferae* include carrot (scientific name: *Daucus carota* subsp. *Sativus).* Examples of the plant of the family *Euphorbiaceae* include cassava (scientific name: *Manihot esculenta*)*.* Examples of the plant of the family *Solanaceae* include red pepper (scientific name: *Capsicum annuum)* and tomato (scientific name: *Solanum lycopersicum).* Examples of the plant of the family *Bromeliaceae* include pineapple (scientific name: *Ananas comosus).* Examples of the plant of the family *Caricaceae* include papaya (scientific name: *Carica papaya).* Examples of the plant of the family *Rosaceae* include apple (scientific name: *Malus domestica),* wild cherry (scientific name: *Prunus avium),* and *peach* (scientific name: *Prunus persica*)*.* Examples of the plant of the family *Amaranthaceae* include quinua (scientific name: *Chenopodium quinoa),* spinach (scientific name: *Spinacia oleracea),* and beet (scientific name: *Beta vulgaris* subsp. *vulgaris).* Examples of the plant of the family *Vitaceae* include common grape (scientific name: *Vitis vinifera).* Examples of the plant of the family *Leguminosae* include pigeon pea (scientific name: *Cajanus cajan),* adzuki bean (scientific name: *Vigna angularis),* wild soybean (scientific name: *Glycine soja),* and soybean (scientific name: *Glycine max).* Examples of the plant of the family *Lythraceae* include pomegranate (scientific name: *Punica granatum).* Examples of the plant of the family *Betulaceae* include white birch (scientific name: *Betula platyphylla).* The allergy can produce an allergic reaction upon contact with an antigen or consumption of the antigen. Here, the contact refers to touch to an object and, particularly, as for the human body, refers to attachment to the skin, the mucosa (eyes, lips, etc.) or the like. The consumption refers to incorporation into the body and refers to incorporation by inhalation or through an oral route. In general, allergic reactions caused by consumption of foods are particularly referred to as food allergies. In a preferred mode, the allergy may be a food allergy.

As referred to herein, the "allergy to soybean" refers to the state in which an individual has an allergic reaction caused by proteins, etc. present in soybean which act as an antigen.

As referred to herein, the "antigen" refers to a substance that provokes an allergic reaction, and is also referred to as an "allergen component". The antigen is preferably a protein.

As referred to herein, the "protein" refers to a molecule having a structure in which naturally occurring amino acids are joined together by peptide bond. The number of amino acids present in a protein is not particularly limited. As referred to herein, the term "polypeptide" also means a molecule having a structure in which naturally occurring amino acids are joined together by peptide bond. The number of amino acids present in a polypeptide is not particularly limited. The "polypeptide" conceptually includes the "protein". Also, polypeptides having about 2 to 50 amino acids joined together by peptide bond are in some cases called "peptides", especially. In the case where amino acids can form different enantiomers, the amino acids are understood to form an L-enantiomer, unless otherwise indicated. The amino acid sequences of proteins, polypeptides, or peptides as used herein are represented by one-letter symbols of amino acids in accordance with standard usage and the notational convention commonly used in the art. The leftward direction represents the amino-terminal direction, and the rightward direction represents the carboxy-terminal direction. In the one-letter symbols of amino acids, X can be any substance having an amino group and a carboxyl group that can bind to amino acids at both ends, and particularly represents that any of 20 types of naturally occurring amino acids are acceptable.

### Identification of antigens

Proteins contained in soybean were analyzed by the aforementioned technique to identify causative antigens of an allergy to soybean. To be specific, soybean proteins were subjected to two-dimensional electrophoresis under the conditions described below.

The electrophoresis in the first dimension was isoelectric focusing, which was performed using isoelectric focusing gels with a gel-strip length of 5 to 10 cm and a gel pH range of 3 to 10. The pH gradient of the gels in the direction of electrophoresis was as follows: when the total gel-strip length is taken as 1, the gel-strip length up to pH 5 is taken as "a", the gel-strip length from pH 5 to 7 is taken as "b", and the gel-strip length above pH 7 is taken as "c", "a" is in the range of 0.15 to 0.3, "b" is in the range of 0.4 to 0.7, and "c" is in the range of 0.15 to 0.3. More specifically, the isoelectric focusing was performed using the IPG gels, Immobiline Drystrip (pH3-10NL), produced by GE Healthcare Bio-Sciences Corporation (hereinafter abbreviated as "GE"). The electrophoresis system used was IPGphor produced by GE. The maximum current of the electrophoresis system was limited to 75 µA per gel strip. The voltage program adopted to perform the first-dimensional isoelectric focusing was as follows: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

The electrophoresis in the second dimension was SDS-PAGE, which was performed using polyacrylamide gels whose gel concentration at the distal end in the direction of electrophoresis was set to 3 to 6% and whose gel concentration at the proximal end was set to a higher value than that at the distal end. More specifically, the SDS-PAGE was performed using NuPAGE 4-12% Bris-Tris Gels (IPG well, Mini, 1 mm) produced by Life Technologies. The electrophoresis system used was XCell SureLock Mini-Cell produced by Life Technologies. The electrophoresis was run at a constant voltage of 200 V for about 45 minutes using an electrophoresis buffer composed of 50 mM MOPS, 50 mM Tris base, 0.1% (w/v) SDS and 1mM EDTA.

As a result, the following spots in a two-dimensional electrophoresis gel run under the conditions described above for proteins in soybean have been revealed to exhibit specific binding to IgE antibodies from soybean-allergic patients.
Spot 1α: Molecular weight 70 to 120 kDa, pI 2.5 to 6.5
Spot 2α: Molecular weight 50 to 120 kDa, pI 2.5 to 6.5
Spot 4α: Molecular weight 70 to 120 kDa, pI 3.5 to 7.5
Spot 5α: Molecular weight 70 to 170 kDa, pI 3.5 to 7.5
Spot 6α: Molecular weight 70 to 120 kDa, pI 3.5 to 7.5
Spot 7α: Molecular weight 50 to 120 kDa, pI 3.5 to 7.5
Spot 8α: Molecular weight 50 to 120 kDa, pI 3.5 to 8.5
Spot 9α: Molecular weight 50 to 120 kDa, pI 3.5 to 8.5
Spot 10α: Molecular weight 30 to 90 kDa, pI 3.5 to 7.5
Spot 11α: Molecular weight 30 to 90 kDa, pI 3.5 to 8.5
Spot 12α: Molecular weight 30 to 90 kDa, pI 3.5 to 8.5
Spot 13α: Molecular weight 30 to 90 kDa, pI 3.5 to 7.5
Spot 14α: Molecular weight 30 to 90 kDa, pI 2.5 to 6.5
Spot 15α, 16α: Molecular weight 20 to 90 kDa, pI 3.5 to 7.5
Spot 17α: Molecular weight 20 to 90 kDa, pI 3.5 to 7.5
Spot 18α: Molecular weight 30 to 90 kDa, pI 3.5 to 8.5
Spot 19α: Molecular weight 30 to 90 kDa, pI 3.5 to 8.5
Spot 25α: Molecular weight 20 to 90 kDa, pI 3.5 to 7.5
Spot 26α: Molecular weight 20 to 90 kDa, pI 3.5 to 7.5
Spot 27α: Molecular weight 20 to 90 kDa, pI 3.5 to 7.5
Spot 28α: Molecular weight 10 to 70 kDa, pI 3.5 to 7.5
Spot 29α, 30α, 31α, 32α, 33α: Molecular weight 10 to 70 kDa, pI 3.5 to 8.5
Spot 34α: Molecular weight 10 to 70 kDa, pI 5.5 to 10.5
Spot 35α: Molecular weight 20 to 90 kDa, pI 5.5 to 10.5
Spot 36α, 37α: Molecular weight 10 to 70 kDa, pI 3.5 to 8.5
Spot 38α: Molecular weight 70 to 170 kDa, pI 5.5 to 10.5
Spot 39α: Molecular weight 5 to 60 kDa, pI 5.5 to 10.5
Spot 40α, 56α: Molecular weight 5 to 60 kDa, pI 5.5 to 10.5
Spot 41α: Molecular weight 5 to 60 kDa, pI 5.5 to 10.5
Spot 42α, 43α: Molecular weight 5 to 60 kDa, pI 5.5 to 10.5
Spot 45α: Molecular weight 2 to 50 kDa, pI 3.5 to 7.5
Spot 46α: Molecular weight 2 to 50 kDa, pI 3.5 to 7.5
Spot 47α: Molecular weight 5 to 50 kDa, pI 3.5 to 7.5
Spot 48α: Molecular weight 2 to 50 kDa, pI 3.5 to 8.5
Spot 50α: Molecular weight 2 to 50 kDa, pI 2.5 to 6.5
Spot 51α: Molecular weight 5 to 60 kDa, pI 2.5 to 6.5
Spot 52α: Molecular weight 10 to 70 kDa, pI 3.5 to 7.5
Spot 53α: Molecular weight 5 to 60 kDa, pI 3.5 to 8.5
Spot 54α: Molecular weight 5 to 60 kDa, pI 3.5 to 8.5
Spot 55α: Molecular weight 30 to 90 kDa, pI 5.5 to 10.5

### Antigen

### (35α) Antigen in spot 35α

As the result of sequence identification of the antigen in spot 35α by mass spectroscopy, the amino acid sequences of SEQ ID NOs: 461-477 were detected.

Also, the mass spectroscopic data obtained for spot 35α on a mass spectrometer was analyzed by comparing the data against the NCBI protein data, and as a result, elongation factor 1-alpha (amino acid sequence: SEQ ID NO: 460, encoding nucleotide sequence: SEQ ID NO: 459) was identified as a protein comprising any of SEQ ID NOs: 461 to 466, elongation factor 1-alpha (amino acid sequence: SEQ ID NO: 468, encoding nucleotide sequence: SEQ ID NO: 467) was identified as a protein comprising any of SEQ ID NOs: 469 to 471, and elongation factor-1A isoform X1 (amino acid sequence: SEQ ID NO: 473, encoding nucleotide sequence: SEQ ID NO: 472) was identified as a protein comprising any of SEQ ID NOs: 474 to 477.

Accordingly, the antigen in spot 35α in the present invention can be any of proteins selected from the group consisting of the proteins as defined below in (35αA1-a) to (35αA1-e), (35αB1), (35αA2-a) to (35αA2-e), (35αB2), (35αA3-a) to (35αA3-e), and (35αB3):
(35αA1-a) a protein comprising an amino acid sequence with deletion, substitution, insertion or addition of one or several amino acids in SEQ ID NO: 460;
(35αA1-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 460;
(35αA1-c) a protein comprising an amino acid sequence encoded by a nucleotide sequence with deletion, substitution, insertion or addition of one or several nucleotides in SEQ ID NO: 459;
(35αA1-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 459;
(35αA1-e) a protein comprising an amino acid sequence encoded by a nucleic acid that hybridizes under stringent conditions with a nucleic acid having a nucleotide sequence complementary to the nucleotide sequence of SEQ ID NO: 459;
(35αB1) a protein comprising at least one amino acid sequence selected from the group consisting of SEQ ID NOs: 461-466, preferably a protein comprising at least 2, 3, 4, or 5 or all sequences of the amino acid sequences. As referred to above, the amino acid sequence of any of SEQ ID NOs: 412 to 416 may be an amino acid sequence derived therefrom by deletion, substitution, insertion or addition of one or several amino acids;
(35αA2-a) a protein comprising an amino acid sequence with deletion, substitution, insertion or addition of one or several amino acids in SEQ ID NO: 468;
(35αA2-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 468;
(35αA2-c) a protein comprising an amino acid sequence encoded by a nucleotide sequence with deletion, substitution, insertion or addition of one or several nucleotides in SEQ ID NO: 467;
(35αA2-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 467;
(35αA2-e) a protein comprising an amino acid sequence encoded by a nucleic acid that hybridizes under stringent conditions with a nucleic acid having a nucleotide sequence complementary to the nucleotide sequence of SEQ ID NO: 467;
(35αB2) a protein comprising at least one amino acid sequence selected from the group consisting of SEQ ID NOs: 469-471, preferably a protein comprising at least 2, or all sequences of the amino acid sequences. As referred to above, the amino acid sequence of any of SEQ ID NOs: 469 to 471 may be an amino acid sequence derived therefrom by deletion, substitution, insertion or addition of one or several amino acids.
(35αA3-a) a protein comprising an amino acid sequence with deletion, substitution, insertion or addition of one or several amino acids in SEQ ID NO: 473;
(35αA3-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 473;
(35αA3-c) a protein comprising an amino acid sequence encoded by a nucleotide sequence with deletion, substitution, insertion or addition of one or several nucleotides in SEQ ID NO: 472;
(35αA3-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 472;
(35αA3-e) a protein comprising an amino acid sequence encoded by a nucleic acid that hybridizes under stringent conditions with a nucleic acid having a nucleotide sequence complementary to the nucleotide sequence of SEQ ID NO: 472;
(35αB3) a protein comprising at least one amino acid sequence selected from the group consisting of SEQ ID NOs: 474-477, preferably a protein comprising at least 2, 3, or all sequences of the amino acid sequences. As referred to above, the amino acid sequence of any of SEQ ID NOs: 474 to 477 may be an amino acid sequence derived therefrom by deletion, substitution, insertion or addition of one or several amino acids.

The protein selected from the group consisting of the proteins as defined above in (35αA1-a) to (35αA1-e), (35αB1), (35αA2-a) to (35αA2-e), (35αB2), (35αA3-a) to (35αA3-e), and (35αB3) can be proteins that are found in a protein spot with a molecular weight of around 20 to 90 kDa, preferably around 30 to 80 kDa, and an isoelectric point of 5.5 to 10.5, preferably 6.0 to 10.0 on gels used in the two-dimensional electrophoresis performed under the conditions described above in the subsection titled "Identification of antigens".

Epitopes of the aforementioned antigens (35αA1-a) to (35αA1-e) and (35αB1) reside in amino acids 31-45 (SEQ ID NO: 849), amino acids 121-135 (SEQ ID NO: 850), amino acids 192-220 (SEQ ID NO: 853), amino acids 216-225 (SEQ ID NO: 856) of SEQ ID NO: 460. When the antigen in spot 35α contains a variation in the amino acid sequence of SEQ ID NO: 460, amino acids at positions corresponding to the region of at least one of these epitopes may retain the sequence in SEQ ID NO: 460.

From the viewpoint of improving sensitivity (a degree to which a patient can be diagnosed as being positive) or specificity (a degree to which a healthy subject is not diagnosed as being positive), the antigen in spot 35α may be the following variant.

In the epitope having the sequence of SEQ ID NO: 849, sites of amino acids other than X in SEQ ID NO: 847 or 848 are important for binding to IgE antibodies from allergic patients. Thus, in another preferred mode, when the antigen in spot 35α contains a variation in the amino acid sequence of SEQ ID NO: 460, amino acids 31-45 of SEQ ID NO: 460 in this antigen may have the amino acid sequence of SEQ ID NO: 847 or 848.

In the epitope having the sequence of SEQ ID NO: 853, sites of amino acids other than X in SEQ ID NO: 851 or 852 are important for binding to IgE antibodies from allergic patients. Thus, in another preferred mode, when the antigen in spot 35α contains a variation in the amino acid sequence of SEQ ID NO: 460, amino acids 192-220 of SEQ ID NO: 460 in this antigen may have the amino acid sequence of SEQ ID NO: 851 or 852.

In the epitope having the sequence of SEQ ID NO: 856, sites of amino acids other than X in SEQ ID NO: 854 or 855 are important for binding to IgE antibodies from allergic patients. Thus, in another preferred mode, when the antigen in spot 35α contains a variation in the amino acid sequence of SEQ ID NO: 460, amino acids 216-225 of SEQ ID NO: 460 in this antigen may have the amino acid sequence of SEQ ID NO: 854 or 855.

Preferably, a protein that is an antigen in spot 35α has activity equivalent to that of the protein having the amino acid sequence of SEQ ID NO: 460 or 468 (elongation factor 1-alpha) or the protein having the amino acid sequence of SEQ ID NO: 473 (elongation factor-1A isoform X1) or causative of an allergy to soybean.

### (38α) Antigen in spot 38α

As the result of sequence identification of the antigen in spot 38α by mass spectroscopy, the amino acid sequences of SEQ ID NOs: 496-513 were detected.

Also, the mass spectroscopic data obtained for spot 38α on a mass spectrometer was analyzed by comparing the data against the NCBI protein data, and as a result, seed biotin-containing protein SBP65-like isoform X1 (amino acid sequence: SEQ ID NO: 495, encoding nucleotide sequence: SEQ ID NO: 494) was identified as a protein comprising any of SEQ ID NOs: 496 to 513.

Accordingly, the antigens in spot 38α in the present invention can be any protein selected from the group consisting of the proteins as defined below in (38αA-a) to (38αA-e) and (38αB):
(38αA-a) a protein comprising an amino acid sequence with deletion, substitution, insertion or addition of one or several amino acids in SEQ ID NO: 495;
(38αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 495;
(38αA-c) a protein comprising an amino acid sequence encoded by a nucleotide sequence with deletion, substitution, insertion or addition of one or several nucleotides in SEQ ID NO: 494;
(38αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 494;
(38αA-e) a protein comprising an amino acid sequence encoded by a nucleic acid that hybridizes under stringent conditions with a nucleic acid having a nucleotide sequence complementary to the nucleotide sequence of SEQ ID NO: 494;
(38αB) a protein comprising at least one amino acid sequence selected from the group consisting of SEQ ID NOs: 496-513, preferably a protein comprising at least 2, 3, 4, 5, 6, 7, 8, 9, 10, 11, 12, 13, 14, 15, 16 or 17 or all sequences of the amino acid sequences. As referred to above, the amino acid sequence of any of SEQ ID NOs: 496-513 may be an amino acid sequence derived therefrom by deletion, substitution, insertion or addition of one or several amino acids.

The proteins selected from the group consisting of (38αA-a) to (38αA-e) and (38αB) as defined above can be proteins that are found in a protein spot with a molecular weight of around 70 to 170 kDa, preferably around 80 to 160 kDa and an isoelectric point of 5.5 to 10.5, preferably 6.0 to 10.0 on gels used in the two-dimensional electrophoresis performed under the conditions described above in the subsection titled "Identification of antigens".

Epitopes of the antigen in spot 38α reside in amino acids 440-446 or 574-580 (SEQ ID NO: 864), amino acids 71-80 (SEQ ID NO: 867), amino acids 521-535 (SEQ ID NO: 869), amino acids 1-15 (SEQ ID NO: 871), amino acids 31-45 (SEQ ID NO: 874), amino acids 221-235 (SEQ ID NO: 877), and amino acids 461-470 (SEQ ID NO: 882), and amino acids 481-490 (SEQ ID NO: 884), and amino acids 821-835 (SEQ ID NO: 887), and amino acids 861-870 (SEQ ID NO: 889), and amino acids 1031-1040 (SEQ ID NO: 892) of SEQ ID NO: 495. The antigen in spot 38α may retain the sequence in SEQ ID NO: 495 as to amino acids at positions corresponding to the region of at least one of these epitopes.

From the viewpoint of improving sensitivity (a degree to which a patient can be diagnosed as being positive) or specificity (a degree to which a healthy subject is not diagnosed as being positive), the antigen in spot 38α may be the following variant.

In the epitope having the sequence of SEQ ID NO: 864, sites of amino acids other than X in SEQ ID NO: 862 are important for binding to IgE antibodies from allergic patients. Thus, in another preferred mode, amino acids 440-446 and / or 574-580 of SEQ ID NO: 495 in the antigen in spot 38α may have the amino acid sequence of SEQ ID NO: 862.

In the epitope having the sequence of SEQ ID NO: 867, sites of amino acids other than X in SEQ ID NO: 865 or 866 are important for binding to IgE antibodies from allergic patients. Thus, in another preferred mode, amino acids 71-80 of SEQ ID NO: 495 in the antigen in spot 38α may have the amino acid sequence of SEQ ID NO: 865 or 866.

In the epitope having the sequence of SEQ ID NO: 869, sites of amino acids other than X in SEQ ID NO: 868 are important for binding to IgE antibodies from allergic patients. Thus, in another preferred mode, amino acids 521-535 of SEQ ID NO: 495 in the antigen in spot 38α may have the amino acid sequence of SEQ ID NO: 868.

In the epitope having the sequence of SEQ ID NO: 871, sites of amino acids other than X in SEQ ID NO: 870 are important for binding to IgE antibodies from allergic patients. Thus, in another preferred mode, amino acids 1-15 of SEQ ID NO: 495 in the antigen in spot 38α may have the amino acid sequence of SEQ ID NO: 870.

In the epitope having the sequence of SEQ ID NO: 874, sites of amino acids other than X in SEQ ID NO: 872 or 873 are important for binding to IgE antibodies from allergic patients. Thus, in another preferred mode, amino acids 31-45 of SEQ ID NO: 495 in the antigen in spot 38α may have the amino acid sequence of SEQ ID NO: 872 or 873.

In the epitope having the sequence of SEQ ID NO: 877, sites of amino acids other than X in SEQ ID NO: 875 or 876 are important for binding to IgE antibodies from allergic patients. Thus, in another preferred mode, amino acids 221-235 of SEQ ID NO: 495 in the antigen in spot 38α may have the amino acid sequence of SEQ ID NO: 875 or 876.

In the epitope having the sequence of SEQ ID NO: 880, sites of amino acids other than X in SEQ ID NO: 878 or 879 are important for binding to IgE antibodies from allergic patients. Thus, in another preferred mode, amino acids 311-325 of SEQ ID NO: 495 in the antigen in spot 38α may have the amino acid sequence of SEQ ID NO: 878 or 879.

In the epitope having the sequence of SEQ ID NO: 882, sites of amino acids other than X in SEQ ID NO: 881 are important for binding to IgE antibodies from allergic patients. Thus, in another preferred mode, amino acids 461-470 of SEQ ID NO: 495 in the antigen in spot 38α may have the amino acid sequence of SEQ ID NO: 881.

In the epitope having the sequence of SEQ ID NO: 884, sites of amino acids other than X in SEQ ID NO: 883 are important for binding to IgE antibodies from allergic patients. Thus, in another preferred mode, amino acids 481-490 of SEQ ID NO: 495 in the antigen in spot 38α may have the amino acid sequence of SEQ ID NO: 883.

In the epitope having the sequence of SEQ ID NO: 887, sites of amino acids other than X in SEQ ID NO: 885 or 886 are important for binding to IgE antibodies from allergic patients. Thus, in another preferred mode, amino acids 821-835 of SEQ ID NO: 495 in the antigen in spot 38α may have the amino acid sequence of SEQ ID NO: 885 or 886.

In the epitope having the sequence of SEQ ID NO: 889, sites of amino acids other than X in SEQ ID NO: 888 are important for binding to IgE antibodies from allergic patients. Thus, in another preferred mode, amino acids 861-870 of SEQ ID NO: 495 in the antigen in spot 38α may have the amino acid sequence of SEQ ID NO: 888.

In the epitope having the sequence of SEQ ID NO: 892, sites of amino acids other than X in SEQ ID NO: 890 or 891 are important for binding to IgE antibodies from allergic patients. Thus, in another preferred mode, amino acids 1031-1040 of SEQ ID NO: 495 in the antigen in spot 38α may have the amino acid sequence of SEQ ID NO: 890 or 891.

Preferably, a protein that is an antigen in spot 38α has activity equivalent to that of the protein having the amino acid sequence of SEQ ID NO: 495 (seed biotin-containing protein SBP65-like isoform X1) or causative of an allergy to soybean.

### (48α) Antigen in spot 48α

As the result of sequence identification of the antigen in spot 48α by mass spectroscopy, the amino acid sequences of SEQ ID NOs: 566-570 were detected.

Also, the mass spectroscopic data obtained for spot 48α on a mass spectrometer was analyzed by comparing the data against the NCBI protein data, and as a result, uncharacterized protein LOC100306570 (amino acid sequence: SEQ ID NO: 565, encoding nucleotide sequence: SEQ ID NO: 564) was identified as a protein comprising any of SEQ ID NOs: 566 to 570.

Accordingly, the antigens in spot 48α in the present invention can be any protein selected from the group consisting of the proteins as defined below in (48αA-a) to (48αA-e) and (48αB):
(48αA-a) a protein comprising an amino acid sequence with deletion, substitution, insertion or addition of one or several amino acids in SEQ ID NO: 565;
(48αA-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 565;
(48αA-c) a protein comprising an amino acid sequence encoded by a nucleotide sequence with deletion, substitution, insertion or addition of one or several nucleotides in SEQ ID NO: 564;
(48αA-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 564;
(48αA-e) a protein comprising an amino acid sequence encoded by a nucleic acid that hybridizes under stringent conditions with a nucleic acid having a nucleotide sequence complementary to the nucleotide sequence of SEQ ID NO: 564;
(48αB) a protein comprising at least one amino acid sequence selected from the group consisting of SEQ ID NOs: 566-570, preferably a protein comprising at least 2, 3 or 4 or all sequences of the amino acid sequences. As referred to above, the amino acid sequence of any of SEQ ID NOs: 566-570 may be an amino acid sequence derived therefrom by deletion, substitution, insertion or addition of one or several amino acids.

The proteins selected from the group consisting of (48αA-a) to (48αA-e) and (48αB) as defined above can be proteins that are found in a protein spot with a molecular weight of around 2 to 50 kDa, preferably around 5 to 40 kDa and an isoelectric point of 3.5 to 8.5, preferably 4.0 to 8.0 on gels used in the two-dimensional electrophoresis performed under the conditions described above in the subsection titled "Identification of antigens".

Epitopes of the antigen in spot 48α reside in amino acids 94-102 (SEQ ID NO: 921) and amino acids
131-145 (SEQ ID NO: 922) of SEQ ID NO: 565. The antigen in spot 48α may retain the sequence in SEQ ID NO:
565 as to amino acids at positions corresponding to the region of these epitopes.

From the viewpoint of improving sensitivity (a degree to which a patient can be diagnosed as being positive) or specificity (a degree to which a healthy subject is not diagnosed as being positive), the antigen in spot 48α may be the following variant.

In the epitope having the sequence of SEQ ID NO: 921, sites of amino acids other than X in SEQ ID NO: 919 or 918 are important for binding to IgE antibodies from allergic patients. Thus, in another preferred mode, amino acids 94-102 of SEQ ID NO: 565 in the antigen in spot 48α may have the amino acid sequence of SEQ ID NO: 919 or 918.

Preferably, a protein that is an antigen in spot 48α has activity equivalent to that of the protein having the amino acid sequence of SEQ ID NO: 565 (uncharacterized protein LOC100306570) or causative of an allergy to soybean.

The proteins that are the aforementioned antigens (35α), (38α) or (48α) include those proteins whose amino acid residues are modified by phosphorylation, sugar chain modification, aminoacylation, ring-opening, deamination or the like.

By stating herein "deletion, substitution, insertion or addition of one or several amino acids" in relation to amino acid sequence, it is meant that in an amino acid sequence of interest, one or several amino acids (e.g., 30%, preferably 25%, 20%, 15%, 10%, 5%, 3%, 2% or 1%, of amino acids with respect to the total length of the amino acid sequence, or e.g., 1, 2, 3, 4, 5, 6, 7, 8, 9 or 10 amino acids) are deleted, one or several amino acids are substituted by any other amino acids, any other amino acids are inserted, and/or any other amino acids are added.

Among the aforementioned modifications, substitution is preferably conservative substitution. The "conservative substitution" refers to the substitution of a certain amino acid residue by a different amino acid residue having similar physicochemical characteristics, and can be any type of substitution as long as it does not substantially change the characteristics of the structure of the original sequence for example, any type of substitution is acceptable as long as any substituted amino acids do not disrupt the helical structure of the original sequence or other secondary structures that characterize the original sequence. The following gives examples of separate groups of amino acid residues that are conservatively substitutable with each other, but substitutable amino acid residues are not limited to the examples given below.
Group A: leucine, isoleucine, valine, alanine, methionine
Group B: aspartic acid, glutamic acid
Group C: asparagine, glutamine
Group D: lysine, arginine
Group E: serine, threonine
Group F: phenylalanine, tyrosine

In the case of non-conservative substitution, one member belonging to one of the aforementioned groups can be replaced with a member belong to any other group. For example, in order to eliminate the possibility of unwanted sugar-chain modification, amino acids of group B, D or E as listed above may be substituted by those of any other group. Also, cysteines may be deleted or substituted by any other amino acids to prevent them from being folded into a protein in its tertiary structure. Also, in order to maintain the balance between hydrophilicity and hydrophobicity or to increase hydrophilicity for the purpose of facilitating synthesis, any amino acids may be substituted in consideration of the hydropathy scales of amino acids, which are a measure of the hydrophilic and hydrophobic properties of amino acids (J. Kyte and R. Doolittle, J. Mol. Biol., Vol.157, p.105-132, 1982).

As referred to herein, the percent identity between two amino acid sequences can be determined by visual inspection and mathematical calculation. Alternatively, the percent identity can be determined using a computer program. Examples of such computer programs include BLAST, FASTA (Altschul, et al., J. Mol. Biol., 215: 403-410 (1990)), and ClustalW. In particular, various conditions (parameters) for identity searches with the BLAST program are described in Altschul, et al. (Nucl. Acids. Res., 25, p.3389-3402, 1997), and are publicly available on the websites of the National Center for Biotechnology Information (NCBI) and DNA Data Bank of Japan (DDBJ) (Altschul, et al., BLAST Manual, Altschul, et al., NCB/NLM/NIH Bethesda, MD 20894). Also, the percent identity can be determined using a genetic information processing software program, such as GENETYX Ver.7 (Genetyx Corporation), DNASIS Pro (Hitachi Software Engineering Co., Ltd.), or Vector NTI (Infomax Inc.).

By stating herein "deletion, substitution, insertion or addition of one or several nucleotides" in relation to nucleotide sequence, it is meant that in a nucleotide sequence of interest, one or several nucleotides (e.g., 30%, preferably 25%, 20%, 15%, 10%, 5%, 3%, 2% or 1%, of nucleotides with respect to the total length of the nucleotide sequence, or e.g., 1, 5, 10, 15, 20, 25 or 30 nucleotides) are deleted, one or several nucleotides are substituted by any other nucleotides, any other nucleotides are inserted, and/or any other nucleotides are added. It is preferable that such a nucleotide deletion, substitution, insertion or addition should not give rise to a frame shift in an amino acid coding sequence.

As referred to herein, the percent identity between two nucleotide sequences can be determined by visual inspection and mathematical calculation. Alternatively, the percent identity can be determined using a computer program. Examples of such sequence comparison computer programs include the BLASTN program, version 2.2.7, available on the website of the National Library of Medicine
(http://www.ncbi.nlm.nih.gov/blast/bl2seq/bls.html) (Altschul, et al. (1990) J. Mol. Biol., 215: 403-10), or the WU-BLAST 2.0 algorithm. Standard default parameter settings for WU-BLAST 2.0 are found and available on the following website: http://blast.wustl.edu.

As referred to herein, "under stringent conditions" means that hybridization takes place under moderately or highly stringent conditions. To be specific, the moderately stringent conditions can be easily determined by those having ordinary skill in the art on the basis of, for example, the length of DNA. Basic conditions are described in Sambrook, et al., Molecular Cloning: A Laboratory Manual, 3rd ed., ch.6-7, Cold Spring Harbor Laboratory Press, 2001. The moderately stringent conditions include hybridization under the conditions of preferably 1×SSC to 6×SSC at 42°C to 55°C, more preferably 1×SSC to 3×SSC at 45°C to 50°C, most preferably 2×SSC at 50°C. In the case of using a hybridization solution containing, for example, about 50% formamide, a temperature around 5 to 15°C lower than the foregoing should be adopted. Washing is also carried out under the conditions of 0.5×SSC to 6×SSC at 40°C to 60°C. In the process of hybridization and washing, generally 0.05% to 0.2% SDS, preferably about 0.1% SDS, may be added. Likewise, the highly stringent conditions can be easily determined by those having ordinary skill in the art on the basis of, for example, the length of DNA. Generally, the highly stringent (high stringent) conditions include hybridization and/or washing at a higher temperature and/or a lower salt concentration than those adopted under the moderately stringent conditions. For example, hybridization is carried out under the conditions of 0.1×SSC to 2×SSC at 55°C to 65°C, more preferably 0.1×SSC to 1×SSC at 60°C to 65°C, most preferably 0.2×SSC at 63°C. Washing is carried out under the conditions of 0.2×SSC to 2×SSC at 50°C to 68°C, more preferably 0.2×SSC at 60 to 65°C.

The residue of X as to the epitopes of the aforementioned antigens (35α), (38α) or (48α) is an amino acid residue at a site where binding activity against IgE antibodies from allergic patients was maintained even after substitution by alanine in alanine scanning described in Example 4. It is well known to those skilled in the art that even when such a site is substituted by any other amino acids, it is highly probable that this binding activity against IgE antibodies is maintained.

Antigens may be obtained by separating and purifying them from soybean using a combination of protein purification methods well known to those skilled in the art. Also, antigens may be obtained by expressing them as recombinant proteins using a genetic recombination technique well known to those skilled in the art and by separating and purifying them using protein purification methods well known to those skilled in the art.

Exemplary protein purification methods include: solubility-based purification methods such as salt precipitation and solvent precipitation; purification methods based on molecular weight difference, such as dialysis, ultrafiltration, gel filtration and SDS-PAGE; charge-based purification methods such as ion exchange chromatography and hydroxylapatite chromatography; specific affinity-based purification methods such as affinity chromatography; purification methods based on hydrophobicity difference, such as reverse-phase high-performance liquid chromatography; and purification methods based on isoelectric point difference, such as isoelectric focusing.

Preparation of a protein by a genetic recombination technique is carried out by preparing an expression vector comprising an antigen-encoding nucleic acid, introducing the expression vector into appropriate host cells by gene transfer or genetic transformation, culturing the host cells under suitable conditions for expression of a recombinant protein, and recovering the recombinant protein expressed in the host cells.

The "vector" refers to a nucleic acid that can be used to introduce a nucleic acid attached thereto into host cells. The "expression vector" is a vector that can induce the expression of a protein encoded by a nucleic acid introduced therethrough. Exemplary vectors include plasmid vectors and viral vectors. Those skilled in the art can select an appropriate expression vector for the expression of a recombinant protein depending on the type of host cells to be used.

The "host cells" refers to cells that undergo gene transfer or genetic transformation by a vector. The host cells can be appropriately selected by those skilled in the art depending on the type of the vector to be used. The host cells can be derived from prokaryotes such as E. coli. When prokaryotic cells like E coli. are used as host cells, the antigen of the present invention may be designed to contain an N-terminal methionine residue in order to facilitate the expression of a recombinant protein in the prokaryotic cells. The N-terminal methionine can be cleaved from the recombinant protein after expression. Also, the host cells may be cells derived from eukaryotes, such as single-cell eukaryotes like yeast, plant cells and animal cells (e.g., human cells, monkey cells, hamster cells, rat cells, murine cells or insect cells) or silkworm.

Gene transfer or genetic transformation of an expression vector into host cells can be carried out as appropriate by following a technique known to those skilled in the art. Those skilled in the art can make possible the expression of a recombinant protein by selecting suitable conditions for the expression of the recombinant protein as appropriate depending on the type of host cells and culturing the host cells under the selected conditions. Then, those skilled in the art can homogenize the host cells having the expressed recombinant protein, and separate and purify an antigen expressed as the recombinant protein from the resulting homogenate by using an appropriate combination of such protein purification methods as mentioned above.

### Diagnosis kit and method (1)

The present invention provides a method for providing an indicator for diagnosing an allergy to a soybean in a subject, the method comprising the steps of:
(i) contacting a sample obtained from the subject with an antigen, wherein the sample is a solution comprising an Ig antibody;
(ii) detecting binding between an IgE antibody present in the sample from the subject and the antigen; and
(iii) when the binding between the IgE antibody in the subject and the antigen is detected, an indicator of the fact that the subject is allergic to a soybean is provided;
wherein the antigen is at least one of proteins as defined above in any of (35α), (38α) or (48α).

The sample obtained from a subject is a solution containing an Ig antibody, preferably an IgE antibody, as collected from the subject. Examples of such solutions include blood, saliva, sputum, snivel, urine, sweat, and tear. The sample obtained from the subject may be subjected to pretreatment for increasing the concentration of an Ig antibody in the sample before being contacted with an antigen. The pretreatment of a sample may involve, for example, collection of the serum or the plasma from the blood. Furthermore, a Fab moiety that is an antigen-binding moiety may be purified. In a particularly preferred mode, the step (i) mentioned above is carried out by contacting an IgE antibody present in the serum obtained from a subject with an antigen.

The IgE antibody may be the IgE antibody itself or may be mast cells or the like bound to IgE antibodies.

Detection of contact and binding between the sample obtained from a subject and an antigen can be carried out by using a known method. Examples of such methods that can be used include detection by ELISA (Enzyme-Linked Immunosorbent Assay), sandwich immunoassay, immunoblotting, immunoprecipitation, or immunochromatography. These are all techniques for detecting binding between an antigen and an IgE antibody from a subject by contacting and binding the IgE antibody from a subject with the antigen, allowing an enzymatically labelled secondary antibody to act on the IgE antibody specifically bound to the antigen, and adding an enzyme substrate (generally, chromogenic or luminescent reagent) to detect an enzymatic reaction product. Also, a method for detecting a fluorescently labeled secondary antibody can be used. Alternatively, detection by a measurement method capable of evaluating binding between an antigen and an IgE antibody, such as surface plasmon resonance (SPR), can also be used. A plurality of antigen-specific IgE antibodies may be mixed.

The antigen may be provided as an isolated antigen in a state immobilized on a carrier. In this case, the steps (i) and (ii) mentioned above can be carried out using ELISA, sandwich immunoassay, immunochromatography, surface plasmon resonance, or the like. Also, the step (i) mentioned above can be carried out by contacting the sample obtained from a subject with an antigen-immobilized surface. The isolated antigen may be obtained by separating and purifying it from soybean using a combination of protein purification methods well known to those skilled in the art, or by preparing it using a genetic recombination technique. The IgE antibody from the subject may be used in a state immobilized on a carrier, and binding to the antigen may be detected by the aforementioned technique.

The antigen may be in a state unimmobilized on a carrier. In this case, flow cytometry or the like can be used in the aforementioned steps (i) and (ii), and the presence of the antigen bound to the antibody can be confirmed with laser beam. Examples include a basophil activation test (BAT). Another example includes a histamine release test (HRT) which examines whether histamine is released by further contacting an antigen with blood cells in a sample.

The antigen may be detected by immunoblotting in a state separated by two-dimensional electrophoresis. The two-dimensional electrophoresis is a technique for separating a protein sample by performing isoelectric focusing in the first dimension and performing SDS-PAGE (SDS-polyacrylamide gel electrophoresis) in the second dimension. The conditions for two-dimensional electrophoresis are not particularly limited as long as the conditions permit the separation of the antigen of the present invention. For example, the conditions for two-dimensional electrophoresis as described above in the subsection titled "Identification of antigens" can be adopted. Also, the electrophoresis conditions may be defined by reference to the descriptions in Patent Literatures 1 to 4 mentioned above. For example, two-dimensional electrophoresis can be carried out under the conditions that satisfy at least one selected from the group consisting of the following requirements:
(A) the isoelectric focusing gels used in the first dimension should have a gel-strip length of 5 to 10 cm and a gel pH range of 3 to 10, and the pH gradient of the gels in the direction of electrophoresis should be as follows: where the gel-strip length up to pH 5 is taken as "a", that length from pH 5 to 7 as "b", and that length above pH 7 as "c", the relations "a < b" and "b > c" are satisfied;
(B) in the case of (A), when the total gel-strip length is taken as 1, "a" should be in the range of 0.15 to 0.3, "b" should be in the range of 0.4 to 0.7, and "c" should be in the range of 0.15 to 0.3;
(C) in the first dimensional isoelectric focusing, a constant voltage step should be performed by applying a constant voltage ranging from 100 V to 600 V per gel strip containing a sample, and after the electrophoresis variation width during electrophoresis for 30 minutes falls within the range of 5 µA, a voltage-increasing step should be started at which the voltage is increased from the aforementioned constant voltage;
(D) in the case of (C), the final voltage at the voltage-increasing step should be in the range of 3000 V to 6000 V;
(E) the isoelectric focusing gels used in the first dimension should have a longitudinal gel-strip length of 5 to 10 cm, and the electrophoresis gels used in the second dimension should have a gel concentration at the distal end in the direction of electrophoresis, which is in the range of 3 to 6%; and
(F) in the case of (E), the electrophoresis gels used in the second dimension should have a gel concentration at the proximal end in the direction of electrophoresis, which is set to a higher value than that at the distal end in the direction of electrophoresis.

The aforementioned antigens (35α), (38α) or (48α) are antigens that specifically bind to IgE antibodies from patients with allergy to soybean. Therefore, when binding between an IgE antibody from a subject and the antigen is detected, an indicator of the fact that the subject is allergic to soybean is provided.

The present invention further provides a kit for diagnosing an allergy to a soybean, comprising at least one of the aforementioned antigens (35α), (38α) or (48α). The diagnosis kit of this invention may be used in the aforementioned method for providing an indicator for diagnosing an allergy to a soybean or in a diagnosis method as described later. The diagnosis kit of this invention may comprise not only the at least one of the aforementioned antigens (35α), (38α) or (48α), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

In another mode, the aforementioned diagnosis kit comprises a companion diagnostic agent for an allergy to a soybean. The companion diagnostic agent is used for identifying patients expected to respond to pharmaceutical products or identifying patients having the risk of severe adverse reactions to pharmaceutical products, or for studying the reactivity of pharmaceutical products in order to optimize treatment using the pharmaceutical products. Here, the optimization of treatment includes, for example, determination of dosage and administration, judgment regarding discontinuation of administration, and confirmation of an allergen component that is used to cause immunological tolerance.

The present invention further provides a composition for diagnosing an allergy to a soybean, comprising at least one of the aforementioned antigens (35α), (38α) or (48α). The diagnosis composition of this invention can be used in a diagnosis method as described below. The diagnosis composition of this invention may further comprise a pharmaceutically acceptable carrier and/or additives commonly used with the antigen of this invention depending on the need.

In one mode, the present invention provides a method for diagnosing an allergy to a soybean in a subject, the method comprising:
(i) contacting a sample obtained from the subject with an antigen;
(ii) detecting binding between an IgE antibody present in the sample from the subject and the antigen; and
(iii) when the binding between the IgE antibody in the subject and the antigen is detected, diagnosing the subject as being allergic to a soybean;
wherein the antigen is at least one of proteins as defined above in any of (35α), (38α) or (48α). In this method, the steps (i) and (ii) are performed as described above regarding the corresponding steps of the method for providing an indicator for diagnosing an allergy to a soybean.

In another mode, the present invention provides a method for diagnosing an allergy to a soybean in a subject, the method comprising administering to the subject at least one of the aforementioned antigens (35α), (38α) or (48α). This method may be performed in the form of a skin test characterized by applying the antigen onto the skin. Examples of the skin test include various forms of tests, such as: a prick test in which a diagnosis composition is applied onto the skin and then a tiny prick to such an extent as not to provoke bleeding is made in the skin to allow an antigen to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which an antigen is administered intracutaneously to observe a reaction. If a skin reaction such as swelling occurs in a skin portion to which the antigen has been applied, the subject of interest is diagnosed as having an allergy to a soybean. The amount of the antigen to be applied to the skin in such tests can be, for example, not more than 100 µg per dose.

In the process of allergy diagnosis, a load test aiming to identify an antigen is often adopted. At least one of the aforementioned antigens (35α), (38α) or (48α) can be used as an active ingredient for a load test to diagnose an allergy to a soybean. Here, the antigen protein to be used in the load test may be a protein that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

In yet another mode, the present invention provides at least one of the aforementioned antigens (35α), (38α) or (48α), intended for use in the diagnosis of an allergy to a soybean. This also includes the provision of at least one of the aforementioned antigens (35α), (38α) or (48α) mixed with a known antigen.

In still another mode, the present invention provides use of at least one of the aforementioned antigens (35α), (38α) or (48α) for the production of a diagnostic agent for an allergy to a soybean.

### Pharmaceutical composition and treatment method (1)

The present invention provides a pharmaceutical composition comprising at least one of the aforementioned antigens (35α), (38α) or (48α). In one mode, the aforementioned pharmaceutical composition is used for the treatment and diagnosis of an allergy to a soybean.

The present invention also provides a method for treating an allergy to a soybean, the method comprising administering at least one of the aforementioned antigens (35α), (38α) or (48α) to a patient in need of a treatment for an allergy to a soybean.

In another mode, the present invention provides at least one of the aforementioned antigens (35α), (38α) or (48α), intended for use in the treatment for an allergy to a soybean. In yet another mode, the present invention provides use of at least one of the aforementioned antigens (35α), (38α) or (48α) for the production of a therapeutic agent for an allergy to a soybean.

In the process of allergy treatment, a hyposensitization therapy aiming to induce immunological tolerance by administering an antigen to a patient is often adopted. The at least one of the aforementioned antigens (35α), (38α) or (48α) can be used as an active ingredient for a hyposensitization therapy for an allergy to a soybean. Here, the antigen protein to be used in the hyposensitization therapy may be a protein that has been expressed and purified and may be a protein that has been expressed in food or cooking ingredients, such as rice-based vaccine expressing pollen allergens which are obtained by transforming rice with a gene of a cedar pollen antigen and expressing the antigen protein in the rice.

The pharmaceutical composition of the present invention can be administered by common administration routes. Examples of common administration routes include oral, sublingual, percutaneous, intracutaneous, subcutaneous, intravascular, intranasal, intramuscular, intraperitoneal, and intrarectal administrations.

The pharmaceutical composition of the present invention can be used as a pharmaceutical composition to which a commonly used pharmaceutically acceptable adjuvant or excipient or any other additives (e.g., stabilizer, solubilizer, emulsifier, buffer, preservative, colorant) are added by a conventional method together with the antigen of this invention depending on the need. The dosage form of the pharmaceutical composition can be selected by those skilled in the art as appropriate depending on the administration route. The pharmaceutical composition can be in the form of, for example, tablet, capsule, troche, sublingual tablet, parenteral injection, intranasal spray, poultice, solution, cream, or lotion. The administration dose, frequency and/or period of the pharmaceutical composition of this invention can be selected by a physician as appropriate depending on the administration route and the patient's condition and characteristics such as age and body weight. For example, the pharmaceutical composition may be administered to an adult patient at a dose of not more than 100 µg per dose. The administration interval can be, for example, once daily, once weekly, twice weekly, once per three months or so. The administration period can be, for example, several weeks to several years. The pharmaceutical composition may be administered in such a manner that the dose is increased in incremental steps over the administration period.

### Tester (1)

The present invention provides a tester comprising an antibody for at least one of the aforementioned antigens (35α), (38α) or (48α).

The antibody can be prepared by a conventional method. For example, the antibody may be prepared by immunizing a mammal such as rabbit with one of the aforementioned antigens (35α), (38α) or (48α). The antibody may be an Ig antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and an antigen. Any given carrier known to those skilled in the art can be used.

Examples of a method for determining the presence or absence of an antigen include the following methods:
a method in which a prepared tester comprising an IgE antibody is contacted with a sample obtained from a food, a cooking ingredient, etc., ELISA or the like method is used to detect whether there is a binding between the IgE antibody and an antigen in the sample, and if the binding between the IgE antibody and the antigen is detected, it is determined that the antibody remains in the food, the cooking ingredient, etc. of interest; and
a method in which filter paper is impregnated with a food or a cooking ingredient and reacted with an antibody solution so as to detect an antigen contained therein.

Another mode of the present invention includes a tester for determining the presence or absence of an antigen of an allergy to a soybean in an object of interest, the tester comprising a primer having a nucleotide sequence complementary to a portion of at least one of the nucleotide sequences of SEQ ID NOs: 1, 3, 5, 7, 9, 11, 13, 15, 69, 88, 105, 121, 138, 154, 158, 162, 168, 173, 188, 200, 203, 219, 232, 238, 241, 253, 279, 285, 290, 305, 319, 326, 333, 340, 347, 357, 366, 383, 393, 410, 417, 421, 428, 434, 440, 446, 452, 455, 459, 467, 472, 478, 484, 488, 494, 514, 517, 521, 524, 532, 540, 546, 552, 555, 559, 564, 571, 577, 583, 589, 598, 605, 612, 617 and 636. The primer has, for example, but not limited to, a nucleotide sequence complementary to, preferably, 12 residues, 15 bases, 20 bases, or 25 bases, in a 3'-terminal or central sequence in a partial sequence of at least one of the nucleotide sequences of SEQ ID NOs defined above in this paragraph. Particularly, when mRNA is of interest, the tester has a complementary primer of a poly-A tail. In a preferred mode, the tester comprising the primer mentioned above may further comprise a primer comprising a 5'-terminal nucleotide sequence, preferably a nucleotide sequence of 12 bases, 15 bases, 20 bases, or 25 bases, of at least one of the nucleotide sequences of SEQ ID NOs defined above in this paragraph.

For example, cDNA is amplified by PCR (Polymerase Chain Reaction) including RT-PCR using templated DNA or mRNA obtained from a soybean and the aforementioned complementary primer, and the sequence of the amplified cDNA is compared with the nucleotide sequences of SEQ ID NOs defined above in the previous paragraph to determine the presence or absence of the antigen. Amplification methods by PCR can be exemplified by RACE. In this respect, even if there exists a point mutation encoding the same amino acid in the comparison of the amplified cDNA with the nucleotide sequences of SEQ ID NOs defined above in the previous paragraph, or even if the nucleotide sequence of the amplified cDNA has insertion, deletion, substitution or addition of bases in the nucleotide sequences of SEQ ID NOs defined above in the previous paragraph, it is determined that the antigen is present when the amino acid sequence encoded by the cDNA has at least 70%, preferably at least 80, 90, 95, 98, or 99% identity to the amino acid sequence of SEQ ID NO: 2, 4, 6, 8, 10, 12, 14, 16, 70, 89, 106, 122, 139, 155, 159, 163, 169, 174, 189, 201, 204, 220, 233, 239, 242, 254, 280, 286, 291, 306, 320, 327, 334, 341, 348, 358, 367, 384, 394, 411, 418, 422, 429, 435, 441, 447, 453, 456, 460, 468, 473, 479, 485, 489, 495, 515, 518, 522, 525, 533, 541, 547, 553, 556, 560, 565, 572, 578, 584, 590, 599, 606, 613, 618 or 637.

In one mode, the aforementioned tester is used to determine the presence or absence of an antigen in cooking ingredients or in products of interest in a food production line. The tester may also be used for quality inspection of production lines and pre-shipment products by manufacturers, or may be used for self-checking of the presence or absence of an antigen in a food or cooking ingredients of interest by consumers.

### Allergen-free food and the like (1)

The present invention provides a soybean or processed product of soybean in which at least one of the aforementioned antigens (35α), (38α) or (48α) is eliminated or reduced.

The method for eliminating or reducing the antigen of the present invention in a soybean or processed products of soybean is not limited. The elimination or reduction of the antigen may be conducted by any method, as long as the method permits the elimination or reduction of the antigen.

For example, the soybean of the present invention whose antigen is eliminated or reduced may be obtained by preparing a soybean in which the expression of the antigen of the present invention is knocked out, using a gene knock-out technique. Any technique known to those skilled in the art such as genetic modification can be used as the gene knock-out technique.

An antigen of the present invention may be the artefact that an antigen of the present invention assumed the removal or a reduced soybean raw material as for the removal or the reduced processed products of soybean. In the case of using an ordinary soybean as a source ingredient, a treatment for removing or reducing the antigen of this invention is performed before or after preparation of a processed product of soybean. The methods for removing or reducing the antigen of the present invention in the processed products of soybean which assumed an ordinary soybean raw material include a method to remove protein component in food such as a high pressure treatment and elution with the neutral salt solution or the high temperature steam, and a method to perform hydrolysis, denaturation, or amino acid alteration (chemical modification, elimination, or the like of a side chain) by heat treatment and acid treatment.

### Method for producing antigen-free processed product of soybean

The present invention provides a method for producing a processed product of soybean in which an antigen is eliminated or reduced, the method comprising the step of confirming that the antigen is eliminated or reduced, in a production process of the processed product, wherein the antigen is at least one of the aforementioned antigens (35α), (38α) or (48α).

The step of confirming that the antigen is eliminated or reduced, in a production process of the processed product of soybean in which an antigen is eliminated or reduced may be performed by confirming the presence or absence of an antigen by the method described above in the subsection titled "Tester (1)".

The production of the processed product of soybean in which an antigen is eliminated or reduced may be performed by the method described above in the subsection titled "Allergen-free food and the like (1)".

### Epitope of antigen

Epitopes and amino acids important for binding activity against IgE antibodies from allergic patients within the epitopes were identified as shown in Examples 4 and 5 as to antigens identified as shown in Example 3 and known antigens Gly m 4, Gly m 5, Gly m 6 and Gly m 8.

The present invention provides polypeptides of the following (2β) to (60β) as polypeptides comprising an amino acid sequence specifically binding to an IgE antibody from an allergic patient.

### (35β) Epitope of elongation factor 1-alpha (spot 35α)

In the present invention, the polypeptide (35β) can be any polypeptide selected from the group consisting of (35β-1) to (35β-4) as defined below:
(35β-1) a polypeptide comprising at least one amino acid sequence selected from the group consisting of SEQ ID NOs: 849, 850, 853 and 856;
(35β-2) a polypeptide comprising the amino acid sequence of XXXXDKXXXERXXXE (SEQ ID NO: 847), preferably a polypeptide comprising the amino acid sequence of XXXXDKRVXERXXXE (SEQ ID NO: 848), more preferably a polypeptide comprising an amino acid sequence in which any 1, 2, 3, 4, 5, 6, 7, 8, 9 or 10 of the amino acids at positions 1-4, 7-9 and 12-14 of SEQ ID NO: 849 are substituted by alanine, further preferably a polypeptide comprising an amino acid sequence in which any 1, 2, 3, 4, 5, 6, 7 or 8 of the amino acids at positions 1-4, 9 and 12-14 of SEQ ID NO: 849 are substituted by other amino acids;
(35β-3) a polypeptide comprising the amino acid sequence of PISXFEXXX (SEQ ID NO: 851), preferably a polypeptide comprising the amino acid sequence of PISGFEXXX (SEQ ID NO: 852), more preferably a polypeptide comprising an amino acid sequence in which any 1, 2, 3, 4 or 5 of the amino acids at positions 4 and 7-9 of SEQ ID NO: 853 are substituted by alanine, further preferably a polypeptide comprising an amino acid sequence in which any 1, 2 or 3 of the amino acids at positions 7-9 of SEQ ID NO: 853 are substituted by other amino acids;
(35β-4) a polypeptide comprising the amino acid sequence of XXEXLDXXXX (SEQ ID NO: 854), preferably a polypeptide comprising the amino acid sequence of LLEXLDXXXX (SEQ ID NO: 855), more preferably a polypeptide comprising an amino acid sequence in which any 1, 2, 3, 4, 5, 6 or 7 of the amino acids at positions 1, 2, 4 and 7-10 of SEQ ID NO: 856 are substituted by alanine, further preferably a polypeptide comprising an amino acid sequence in which any 1, 2, 3, 4 or 5 of the amino acids at positions 4 and 7-10 of SEQ ID NO: 856 are substituted by other amino acids.

### (38β) Epitope of seed biotin-containing protein SBP65-like isoform X1 (spot 38α)

In the present invention, the polypeptide (38β) can be any polypeptide selected from the group consisting of (38β-1) to (38β-13) as defined below:
(38β-1) a polypeptide comprising at least one amino acid sequence selected from the group consisting of SEQ ID NOs: 864, 867, 869, 871, 874, 877, 880, 882, 884, 887, 889 and 892;
(38β-2) a polypeptide comprising the amino acid sequence of RGKXXXX (SEQ ID NO: 862), preferably a polypeptide comprising an amino acid sequence in which any 1, 2, 3 or 4 of the amino acids at positions 4-7 of SEQ ID NO: 864 are substituted by alanine, further preferably a polypeptide comprising an amino acid sequence in which any 1, 2, 3 or 4 of the amino acids at positions 4-7 of SEQ ID NO: 864 are substituted by other amino acids;
(38β-3) a polypeptide comprising the amino acid sequence of XRGKXXXXXX (SEQ ID NO: 865), preferably a polypeptide comprising the amino acid sequence of RRGKXXXXXX (SEQ ID NO: 866), more preferably a polypeptide comprising an amino acid sequence in which any 1, 2, 3, 4, 5, 6 or 7 of the amino acids at positions 1 and 5-10 of SEQ ID NO: 867 are substituted by alanine, further preferably a polypeptide comprising an amino acid sequence in which any 1, 2, 3, 4, 5 or 6 of the amino acids at positions 5-10 of SEQ ID NO: 867 are substituted by other amino acids;
(38β-4) a polypeptide comprising the amino acid sequence of RXKXXXXXXXXXXXE (SEQ ID NO: 868), preferably a polypeptide comprising an amino acid sequence in which any 1, 2, 3, 4, 5, 6, 7, 8, 9, 10, 11 or 12 of the amino acids at positions 2 and 4-14 of SEQ ID NO: 869 are substituted by alanine, further preferably a polypeptide comprising an amino acid sequence in which any 1, 2, 3, 4, 5, 6, 7, 8, 9, 10, 11 or 12 of the amino acids at positions 2 and 4-14 of SEQ ID NO: 869 are substituted by other amino acids;
(38β-5) a polypeptide comprising the amino acid sequence of XXXXXXXRRXXXXXR (SEQ ID NO: 870), preferably a polypeptide comprising an amino acid sequence in which any 1, 2, 3, 4, 5, 6, 7, 8, 9, 10, 11 or 12 of the amino acids at positions 1-7 and 10-14 of SEQ ID NO: 871 are substituted by alanine, further preferably a polypeptide comprising an amino acid sequence in which any 1, 2, 3, 4, 5, 6, 7, 8, 9, 10, 11 or 12 of the amino acids at positions 1-7 and 10-14 of SEQ ID NO: 871 are substituted by other amino acids;
(38β-6) a polypeptide comprising the amino acid sequence of XXXKGRVVXEXXXXX (SEQ ID NO: 872), preferably a polypeptide comprising the amino acid sequence of LPDKGRVVXESEKKE (SEQ ID NO: 873), more preferably a polypeptide comprising an amino acid sequence in which any 1, 2, 3, 4, 5, 6, 7, 8 or 9 of the amino acids at positions 1-3, 9 and 11-15 of SEQ ID NO: 874 are substituted by alanine, further preferably a polypeptide comprising an amino acid sequence in which any 1, 2, 3, 4, 5, 6, 7, 8 or 9 of the amino acids at positions 1-3, 9 and 11-15 of SEQ ID NO: 874 are substituted by other amino acids;
(38β-7) a polypeptide comprising the amino acid sequence of ATKAKDXXREXXXXX (SEQ ID NO: 875), preferably a polypeptide comprising the amino acid sequence of ATKAKDVTREXXXXX (SEQ ID NO: 876), more preferably a polypeptide comprising an amino acid sequence in which any 1, 2, 3, 4, 5, 6 or 7 of the amino acids at positions 7, 8 and 11-15 of SEQ ID NO: 877 are substituted by alanine, further preferably a polypeptide comprising an amino acid sequence in which any 1, 2, 3, 4 or 5 of the amino acids at positions 11-15 of SEQ ID NO: 877 are substituted by other amino acids;
(38β-8) a polypeptide comprising the amino acid sequence of XGXKGQXKKXXXXXX (SEQ ID NO: 878), preferably a polypeptide comprising the amino acid sequence of XGXKGQTKKXXGEEQ (SEQ ID NO: 879), more preferably a polypeptide comprising an amino acid sequence in which any 1, 2, 3, 4, 5, 6, 7, 8 or 9 of the amino acids at positions 1, 3, 7 and 10-15 of SEQ ID NO: 880 are substituted by alanine, further preferably a polypeptide comprising an amino acid sequence in which any 1, 2, 3 or 4 of the amino acids at positions 1, 3, 10 and 11 of SEQ ID NO: 880 are substituted by other amino acids;
(38β-9) a polypeptide comprising the amino acid sequence of RXXXETKEGX (SEQ ID NO: 881), preferably a polypeptide comprising an amino acid sequence in which any 1, 2, 3 or 4 of the amino acids at positions 2-4 and 10 of SEQ ID NO: 882 are substituted by alanine, further preferably a polypeptide comprising an amino acid sequence in which any 1, 2, 3 or 4 of the amino acids at positions 2-4 and 10 of SEQ ID NO: 882 are substituted by other amino acids;
(38β-10) a polypeptide comprising the amino acid sequence of XXXXNKQSSL (SEQ ID NO: 883), preferably a polypeptide comprising an amino acid sequence in which any 1, 2, 3 or 4 of the amino acids at positions 1-4 of SEQ ID NO: 884 are substituted by alanine, further preferably a polypeptide comprising an amino acid sequence in which any 1, 2, 3 or 4 of the amino acids at positions 1-4 of SEQ ID NO: 884 are substituted by other amino acids;
(38β-11) a polypeptide comprising the amino acid sequence of XKXVAXXXXXXXXEX (SEQ ID NO: 885), preferably a polypeptide comprising the amino acid sequence of XKXVAEXXXQXASEL (SEQ ID NO: 886), more preferably a polypeptide comprising an amino acid sequence in which any 1, 2, 3, 4, 5, 6, 7, 8, 9, 10 or 11 of the amino acids at positions 1, 3, 6-13 and 15 of SEQ ID NO: 887 are substituted by alanine, further preferably a polypeptide comprising an amino acid sequence in which any 1, 2, 3, 4, 5 or 6 of the amino acids at positions 1, 3, 7-9 and 11 of SEQ ID NO: 887 are substituted by other amino acids;
(38β-12) a polypeptide comprising the amino acid sequence of XXKVEAEXXX (SEQ ID NO: 888), preferably a polypeptide comprising an amino acid sequence in which any 1, 2, 3, 4 or 5 of the amino acids at positions 1, 2 and 8-10 of SEQ ID NO: 889 are substituted by alanine, further preferably a polypeptide comprising an amino acid sequence in which any 1, 2, 3, 4 or 5 of the amino acids at positions 1, 2 and 8-10 of SEQ ID NO: 889 are substituted by other amino acids;
(38β-13) a polypeptide comprising the amino acid sequence of XXXXIAEIAE (SEQ ID NO: 890), preferably a polypeptide comprising the amino acid sequence of XXETIAEIAE (SEQ ID NO: 891), more preferably a polypeptide comprising an amino acid sequence in which any 1, 2, 3 or 4 of the amino acids at positions 1-4 of SEQ ID NO: 892 are substituted by alanine, further preferably a polypeptide comprising an amino acid sequence in which any 1 or 2 of the amino acids at positions 1 and 2 of SEQ ID NO: 892 are substituted by other amino acids.

### (48β) Epitope of uncharacterized protein LOC100306570 (spot 48α)

In the present invention, the polypeptide (48β) can be any polypeptide selected from the group consisting of (48β-1) to (48β-2) as defined below:
(48β-1) a polypeptide comprising at least one amino acid sequence selected from the group consisting of SEQ ID NOs: 921 and 922;
(48β-2) a polypeptide comprising the amino acid sequence of XXXXXXFDK (SEQ ID NO: 919), preferably a polypeptide comprising the amino acid sequence of XXXXPXFDK (SEQ ID NO: 920), more preferably a polypeptide comprising an amino acid sequence in which any 1, 2, 3, 4, 5 or 6 of the amino acids at positions 1-6 of SEQ ID NO: 921 are substituted by alanine, further preferably a polypeptide comprising an amino acid sequence in which any 1, 2, 3, 4 or 5 of the amino acids at positions 1-4 and 6 of SEQ ID NO: 921 are substituted by other amino acids.

The residue of X as to the aforementioned polypeptides (2β) to (60β) is an amino acid residue at a site where binding activity against IgE antibodies from allergic patients was maintained even after substitution by alanine in alanine scanning described in Example 4. It is well known to those skilled in the art that even when such a site is substituted by any other amino acids, it is highly probable that this binding activity against IgE antibodies is maintained.

The lengths of the aforementioned polypeptides (2β) to (60β) are not particularly limited. In a preferred mode, the lengths of the aforementioned polypeptides (2β) to (60β) can be at least 500 amino acids, at least 300 amino acids, at least 200 amino acids, at least 100 amino acids, at least 50 amino acids, at least 30 amino acids, at least 20 amino acids, at least 10 amino acids, or at least 5 amino acids.

The aforementioned polypeptides (2β) to (60β) may be prepared by a technique of chemical synthesis such as solid-phase peptide synthesis. Alternatively, polypeptides comprising an epitope may be obtained by expressing them as recombinant proteins using a genetic recombination technique well known to those skilled in the art and by separating and purifying them using protein purification methods well known to those skilled in the art.

### Diagnosis kit and method (2)

The present invention provides a method for providing an indicator for diagnosing an allergy in a subject, the method comprising the steps of:
(i) contacting a sample obtained from the subject with an antigen, wherein the sample is a solution comprising an Ig antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is at least one of the aforementioned polypeptides (2β) to (60β), or a polypeptide in which two or more of the aforementioned polypeptides (2β) to (60β) are joined together via or without a spacer.

Hereinafter, the at least one of the aforementioned polypeptides (2β) to (60β), or the polypeptide in which two or more of the aforementioned polypeptides (2β) to (60β) are joined together via or without a spacer is referred to as the "antigen including (2β) to (60β)" in the present specification. The type of the spacer is not particularly limited, and an ordinary spacer that is used by those skilled in the art for joining together two or more peptides can be used. The spacer may be, for example, a hydrocarbon chain such as Acp(6)-OH.

The sample obtained from a subject is as described above in the subsection titled "Diagnosis kit and method (1)".

Detection of contact and binding between the sample obtained from a subject and the antigen can be carried out by using a known method described above in the subsection titled "Diagnosis kit and method (1)", such as ELISA (Enzyme-Linked Immunosorbent Assay), sandwich immunoassay, immunoblotting, immunoprecipitation, or immunochromatography.

The antigen may be provided as an isolated antigen including (2β) to (60β) in a state immobilized on a carrier. In this case, the steps (i) and (ii) mentioned above can be carried out using ELISA or sandwich immunoassay, or the like. In this respect, the step (i) mentioned above can be carried out by contacting the sample obtained from a subject with an antigen-immobilized surface.

The antigen including (2β) to (60β) specifically binds to IgE antibodies from allergic patients. Therefore, when binding between an IgE antibody from a subject and the antigen is detected, an indicator of the fact that the subject is allergic to soybean is provided.

The present invention further provides a kit for diagnosing an allergy, comprising at least one antigen including (2β) to (60β). The diagnosis kit of this invention may be used in the aforementioned method for providing an indicator for diagnosing an allergy or in a diagnosis method as described later. The diagnosis kit of this invention may comprise not only the at least one antigen including (2β) to (60β), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

The present invention further provides a composition for diagnosing an allergy, comprising at least one antigen including (2β) to (60β). The diagnosis composition of this invention can be used in a diagnosis method as described below. The diagnosis composition of this invention may further comprise a pharmaceutically acceptable carrier and/or additives commonly used with the antigen of this invention depending on the need.

In one mode, the present invention provides a method for diagnosing an allergy in a subject, the method comprising:
(i) contacting a sample obtained from the subject with an antigen;
(ii) detecting binding between an IgE antibody present in the sample obtained from the subject and the antigen; and
(iii) when the binding between the IgE antibody in the subject and the antigen is detected, diagnosing the subject as being allergic to a soybean;
wherein the antigen is at least one of the aforementioned polypeptides (2β) to (60β), or a polypeptide in which two or more of the aforementioned polypeptides (2β) to (60β) are joined together via or without a spacer (i.e., the antigen including (2β) to (60β)). In this method, the steps (i) and (ii) are performed as described above regarding the corresponding steps of the method for providing an indicator for diagnosing an allergy.

In another mode, the present invention provides a method for diagnosing an allergy in a subject, the method comprising administering to the subject at least one antigen including (2β) to (60β). This method may be performed in the form of a skin test characterized by applying the antigen onto the skin. The skin test includes forms of tests described above in the subsection titled "Diagnosis kit and method (1)". If a skin reaction such as swelling occurs in a skin portion to which the antigen has been applied, the subject of interest is diagnosed as having an allergy. The amount of the antigen to be applied to the skin in such tests can be, for example, not more than 100 µg per dose.

In yet another mode, the present invention provides at least one antigen including (2β) to (60β), intended for use as an active ingredient for a load test for allergy diagnosis.

In still another mode, the present invention provides at least one antigen including (2β) to (60β), intended for use in the diagnosis of an allergy.

In still another mode, the present invention provides use of at least one antigen including (2β) to (60β) for the production of a diagnostic agent for an allergy.

In this subsection, the allergy to be diagnosed or detected can be allergies to allergens in which allergen components having the aforementioned polypeptides (2β) to (60β) are present. Thus, detection of the allergy can be detection of not only an allergy to a single allergen, but also allergies including cross-reactivity.

### Pharmaceutical composition and treatment method (2)

The present invention provides a pharmaceutical composition comprising at least one antigen including (2β) to (60β). In one mode, the aforementioned pharmaceutical composition is used for the treatment of an allergy.

The present invention also provides a method for treating an allergy, the method comprising administering at least one antigen including (2β) to (60β) to a patient in need of a treatment for an allergy.

In another mode, the present invention provides at least one antigen including (2β) to (60β), intended for use in the treatment for an allergy. In yet another mode, the present invention provides use of at least one antigen including (2β) to (60β) for the production of a therapeutic agent for an allergy.

In still another mode, the at least one antigen including (2β) to (60β) can be used as an active ingredient for hyposensitization therapy for an allergy. Here, the antigen protein to be used in the hyposensitization therapy may be a polypeptide that has been expressed and purified and may be a polypeptide that has been expressed in food such as rice, such as rice-based vaccine expressing pollen allergens.

The administration route, administration dose, frequency and/or period of the pharmaceutical composition of this invention, and other ingredients to be contained in the pharmaceutical composition, and the dosage form can be as described above in the subsection titled "Pharmaceutical composition and treatment method (1)". In the case of using the antigen including (2β) to (60β), for example, the dose to an adult patient may be a dose of not more than 100 µg per dose.

In this subsection, the allergy to be treated can be allergies to allergens in which allergen components having the aforementioned polypeptides (2β) to (60β) are present. Thus, detection of the allergy can be detection of not only an allergy to a single allergen, but also allergies including cross-reactivity.

### Tester (2)

The present invention provides a tester comprising an antibody for at least one antigen including (2β) to (60β).

The antibody can be prepared by a conventional method. For example, the antibody may be prepared by immunizing a mammal such as rabbit with the aforementioned antigen including (2β) to (60β). The antibody may be an Ig antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and the antigen including (2β) to (60β). Any given carrier known to those skilled in the art can be used.

In one mode, the aforementioned tester can be used for the purposes described above in the subsection titled "Tester (1)".

A method for determining the presence or absence of an antigen is as described above in the subsection titled "Tester (1)".

Another mode of the present invention includes a tester for determining the presence or absence of an antigen of an allergy in an object of interest, the tester comprising a primer appropriate for an epitope. The primer is not limited and may be designed so as to comprise, for example, a portion of the nucleotide sequence of a nucleic acid encoding any of the amino acid sequences defined above in (2β) to (60β), or a complementary strand thereof. Alternatively, the primer may be designed so as to be the nucleotide sequence of a region upstream or downstream of a portion encoding an epitope that is any of the amino acid sequences defined above in (2β) to (60β), in a nucleic acid encoding a protein comprising the epitope, or a complementary strand thereof. Here, the position of the epitope in the full-length sequence of an antigen is as defined above in the subsections titled "Antigen" and "Epitope of antigen". Particularly, when mRNA is of interest, the tester has a complementary primer of a poly-A tail.

For example, DNA is amplified by PCR (Polymerase Chain Reaction) including RT-PCR using templated DNA or mRNA obtained from a sample and the aforementioned primer, and the presence or absence of a nucleic acid encoding the amino acid sequences defined above in (2β) to (60β) in the sequence of the amplified DNA is determined to determine the presence or absence of the antigen. Amplification methods by PCR can be exemplified by RACE.

### Allergen-free food and the like (2)

The present invention provides a food raw material or an edible processed product in which at least one antigen including (2β) to (60β) is eliminated or reduced.

The method for eliminating or reducing the antigen of the present invention in a food raw material or an edible processed product is not limited. The techniques described above in the subsection titled "Allergen-free food and the like (1)" may be used. Elimination or reduction of at least one antigen including (2β) to (60β) may be achieved by eliminating or reducing the whole antigen or may be achieved by cleaving or removing the polypeptides defined in (2β) to (60β) from the antigen protein. The "removal" includes deletion and modification.

The edible processed product in which the antigen of the present invention is eliminated or reduced may be a processed product of the food raw material in which the antigen of the present invention is eliminated or reduced, such as powdered milk obtained with purified amino acids as a raw material. In the case of using an ordinary food raw material, a treatment for removing or reducing the polypeptide of this invention is performed before or after preparation of an edible processed product. The techniques described in the subsection titled "Allergen-free food and the like (1)" may be used as methods for removing or reducing the antigen of the present invention in an edible processed product obtained with an ordinary food raw material.

### Method for producing allergen-free food (2)

The present invention provides a method for producing an edible processed product in which an antigen is eliminated or reduced, the method comprising the step of confirming that the antigen is eliminated or reduced, in a production process of the processed product, wherein the antigen is at least one of the aforementioned polypeptides including (35β), (38β) or (48β) and antigens (35α), (38α) or (48α).

In the production method, elimination or reduction of an antigen means that at least one polypeptides including (35β), (38β) or (48β) is eliminated or reduced, or the polypeptides defined in (35β), (38β) or (48β) are cleaved or removed from the antigen protein.

A technique of confirming that the antigen is eliminated or reduced in the production process of the edible processed product is not particularly limited, and any technique capable of detecting at least one of the aforementioned polypeptides including (35β), (38β) or (48β) and antigens (35α), (38α) or (48α) may be used. For example, the presence or absence of the polypeptide or the antigen in the processed product may be confirmed from the binding activity of an antibody for at least one of the aforementioned polypeptides including (35β), (38β) or (48β)and antigens (35α), (38α) or (48α) against a sample containing a material resulting from the production process of the processed product. Details of such a method are as described above in the subsection titled "Diagnosis kit and method (1)". Thus, in the production method, the "IgE antibody from a subject" described above in the subsection titled "Diagnosis kit and method (1)" is replaced with the "antibody for at least one of the aforementioned antigens (35α), (38α) or (48α)", and the "antigen" described above in the subsection titled "Diagnosis kit and method (1)" is replaced with the "sample containing a material resulting from the production process of the edible processed product". The techniques described above in the subsection titled "Diagnosis kit and method (1)" can be used to confirm that the antigen is eliminated or reduced in the production process of the edible processed product.

### EXAMPLES

The following describes examples of the present invention. The technical scope of this invention is not limited by these examples.

### Example 1: Confirmation of a protein pattern

Proteins contained in soybean were investigated using a two-dimensional electrophoresis method described below.

### Protein extraction

Commercially available dry soybeans (several beans) were powdered. 500 µl of water was added to 50 mg of the powder and shaking extraction was conducted using a vortex mixer at 25°C for 10 minutes. After the shaking extraction, a liquid protein extract was collected.

Thereafter, the precipitation procedure was repeated twice using a 2D-CleanUP Kit (produced by GE). In the first round of precipitation, the collected liquid protein extract was precipitated by adding TCA (trichloroacetic acid) thereto and the precipitated product produced by this procedure (TCA-precipitated product) was collected. In the second round of precipitation, the TCA-precipitated product collected above was further precipitated by adding acetone thereto and the precipitated product (sample) produced by this procedure was collected.

### Preparation of a sample solution

Part of the collected sample (50 µg on a protein weight basis) was dissolved in 150 µL of a DeStreak Rehydration Solution (produced by GE), which is a swelling buffer for first-dimensional isoelectric focusing gels, thereby obtaining a sample solution for first-dimensional isoelectric focusing (sample solution for swelling). The constituents of the DeStreak Rehydration Solution are as mentioned below.
7M thiourea
2M urea
4% (w/v) of CHAPS (3-[(3-cholamidopropyl)dimethylammonio]propanesulfonate)
0.5% (v/v) IPG buffer; produced by GE
Moderate amount of BPB (bromophenol blue)

### Penetration of the sample into first-dimensional isoelectric focusing gels

First-dimensional isoelectric focusing gel strips (Immobiline Drystrip IPG gels (pH3-10NL); produced by GE) were immersed in 140 µL of the foregoing sample solution for first-dimensional isoelectric focusing (sample solution for swelling) and impregnated with the solution at room temperature overnight.

In this example, an IPGphor electrophoresis system produced by GE was used.

An electrophoresis tray was filled with silicone oil. Filter paper moisten with water was positioned at both ends of the gel strips impregnated with the sample, and the gel strips were set in the electrophoresis tray such that the gel strips were covered with silicone oil. Electrodes were placed on the gel strips with the filter paper intervening therebetween.

The maximum current of the isoelectric focusing system was set to 75 µA per gel strip, and the first-dimensional isoelectric focusing was carried out according to the following voltage program: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

### SDS equilibration of isoelectric focusing gels

After the aforementioned first-dimensional isoelectric focusing was done, the gel strips were taken out of the isoelectric focusing system, immersed in an equilibration buffer containing a reducing agent, and shaken at room temperature for 15 minutes. The constituents of the equilibration buffer containing the reducing agent are as mentioned below.
100 mM Tris-HCl (pH 8.0)
6M urea
30% (v/v) glycerol
2% (w/v) SDS
1% (w/v) DTT

Next, the equilibration buffer containing the reducing agent was removed, and then the gel strips were immersed in an equilibration buffer containing an alkylating agent and shaken at room temperature for 15 minutes to obtain SDS-equilibrated gels. The constituents of the equilibration buffer containing the alkylating agent are as mentioned below.
100 mM Tris-HCl (pH 8.0)
6M urea
30% (v/v) glycerol
2% (w/v) SDS
2.5% (w/v) iodoacetamide

### Second-dimensional SDS-PAGE

In this example, the XCell SureLock Mini-Cell electrophoresis system produced by Life Technologies was used. The second-dimensional electrophoresis gels used were NuPAGE 4-12% Bis-Tris Gels produced by Life Technologies. Also, an electrophoresis buffer composed of the following constituents was prepared and used.
50 mM MOPS
50 mM Tris base
0.1% (w/v) SDS
1 mM EDTA

Further, an agarose solution for gel adhesion was used in this example, which was prepared by dissolving 0.5% (w/v) Agarose S (produced by Nippon Gene Co., Ltd.) and a moderate amount of BPB (bromophenol blue) in the electrophoresis buffer.

SDS-PAGE wells were washed well with the electrophoresis buffer, and then the buffer used for the washing was removed. Next, the washed wells were charged with the fully dissolved agarose solution for gel adhesion. Next, the SDS-equilibrated gel strips were immersed in agarose and closely adhered to second-dimensional electrophoresis gels using tweezers. After it was confirmed that agarose was fully fixed with the gels being closely adhered to each other, electrophoresis was performed at a constant voltage of 200 V for about 45 minutes.

### Fluorescent staining of gels

The gels were fluorescently stained with SYPRO Ruby (produced by Life Technologies).

First, an airtight container to be used was washed well in advance with 98% (v/v) ethanol. The electrophoresed second-dimensional electrophoresis gel strips were taken out of the SDS-PAGE system, placed onto the washed airtight container, and treated twice by immersion in 50% (v/v) methanol and 7% (v/v) aqueous solution containing acetic acid for 30 minutes. Then, a further immersion treatment was done for 10 minutes, with the solution being replaced by water. Next, the second-dimensional electrophoresis gel strips were immersed in 40 mL of SYPRO Ruby and shaken at room temperature overnight. Thereafter, the SYPRO Ruby was removed, and then the second-dimensional electrophoresis gel strips were washed with water and shaken in 10% (v/v) methanol and 7% (v/v) aqueous solution containing acetic acid for 30 minutes. Further shaking was done for at least 30 minutes, with the solution being replaced by water.

### Analysis

The second-dimensional electrophoresis gels obtained through the foregoing series of treatments were subjected to fluorescent image scanning on Typhoon 9400 (produced by GE). The results of the two-dimensional electrophoresis are shown in Fig. 1. Molecular weight marker bands are found at the left of the panels. The positions of the bands denote particular molecular weights (in KDa).

### Example 2: Identification of antigens by immunoblotting

Identification of antigens by immunoblotting was carried out by taking all the steps up to the step of "Second-dimensional SDS-PAGE" as described above in Example 1, followed by the steps of "Transfer to membrane", "Immunoblotting" and "Analysis" as described below.

### Transfer to membrane

Transfer to membrane was done using the following transfer system and transfer buffer.
Transfer system: XCell SureLock Mini-Cell and XCell II Blot Module (produced by Life Technologies)
Transfer buffer: NuPAGE Transfer Buffer (X20) (produced by Life Technologies), used in a form diluted 20-fold with milliQ water.

To be specific, proteins in the two-dimensional electrophoresis gels were transferred to a membrane (PVDF membrane) according to the following procedure.
(1) The PVDF membrane was immersed in 100% methanol followed by milliQ water, and then moved into the transfer buffer to hydrophilize the PVDF membrane.
(2) After sponge, filter paper, the gels treated by second-dimensional SDS-PAGE, the hydrophilized PVDF membrane, filter paper, and sponge were put in place in this order, the transfer system was energized at a constant voltage of 30 V for one hour.

### Immunoblotting

Immunoblotting of the membrane was carried out using, as a primary antibody, a serum sample from a patient with a soybean allergy or a serum sample from a healthy subject.

Immunoblotting of the membrane was carried out according to the following procedure.
(1) The transferred membrane was shaken in a 5% skim milk/PBST solution (a PBS buffer containing 0.3% Tween 20 nonionic surfactant) at room temperature for one hour.
(2) The membrane was left to stand in a solution of 3% primary antibody serum in 5% skim milk/PBST at room temperature for one hour.
(3) The membrane was washed with a PBST solution (5 min. × 3 times).
(4) The membrane was left to stand in a 1:2500 dilution of the secondary antibody, anti-human IgE-HRP (horseradish peroxidase), with a 3% skim milk/PBST solution at room temperature for one hour.
(5) The membrane was washed with a PBST solution (5 min. × 3 times).
(6) The membrane was left to stand in Pierce Western Blotting Substrate Plus (produced by Thermo Fisher Scientific) for 5 minutes.

### Analysis

The membrane obtained through the foregoing series of treatments was subjected to fluorescent image scanning on Typhoon 9400 (produced by GE).

The immunoblots obtained with the serum from the soybean-allergic patient were compared with those obtained with the control serum from the healthy subject. By immunoblotting using the serum from soybean-allergic Patient 1α, Patient 2α, Patient 3α, Patient 4α, Patient 5α, Patient 6α, Patient 7α, Patient 8α, and Patient 9α, 56 spots were detected (Figs. 3A to I), which are different from the spots detected when the serum of the healthy subject was used (Fig. 2) and different from those of the known soybean allergen proteins.

The molecular weights and isoelectric points of the 56 spots are as follows.
Spot 1α: Molecular weight 80 to 110 kDa, pI 3.0 to 6.0
Spot 2α: Molecular weight 60 to 110 kDa, pI 3.0 to 6.0
Spot 3α: Molecular weight 100 to 150 kDa, pI 4.0 to 6.0
Spot 4α: Molecular weight 80 to 110 kDa, pI 4.0 to 7.0
Spot 5α: Molecular weight 80 to 160 kDa, pI 4.0 to 7.0
Spot 6α: Molecular weight 80 to 110 kDa, pI 4.0 to 7.0
Spot 7α: Molecular weight 60 to 110 kDa, pI 4.0 to 7.0
Spot 8α: Molecular weight 60 to 110 kDa, pI 4.0 to 8.0
Spot 9α: Molecular weight 60 to 110 kDa, pI 4.0 to 8.0
Spot 10α: Molecular weight 40 to 80 kDa, pI 4.0 to 7.0
Spot 11α: Molecular weight 40 to 80 kDa, pI 4.0 to 8.0
Spot 12α: Molecular weight 40 to 80 kDa, pI 4.0 to 8.0
Spot 13α: Molecular weight 40 to 80 kDa, pI 4.0 to 7.0
Spot 14α: Molecular weight 40 to 80 kDa, pI 3.0 to 6.0
Spot 15α, 16α: Molecular weight 30 to 80 kDa, pI 4.0 to 7.0
Spot 17α: Molecular weight 30 to 80 kDa, pI 4.0 to 7.0
Spot 18α: Molecular weight 40 to 80 kDa, pI 4.0 to 8.0
Spot 19α: Molecular weight 40 to 80 kDa, pI 4.0 to 8.0
Spot 20α: Molecular weight 60 to 75 kDa, pI 6.0 to 8.0
Spot 21α: Molecular weight 40 to 60 kDa, pI 5.0 to 8.0
Spot 22α: Molecular weight 25 to 37 kDa, pI 8.0 to 10.0
Spot 23α: Molecular weight 25 to 37 kDa, pI 7.0 to 9.0
Spot 24α: Molecular weight 5 to 20 kDa, pI 5.0 to 7.0
Spot 25α: Molecular weight 30 to 80 kDa, pI 4.0 to 7.0
Spot 26α: Molecular weight 30 to 80 kDa, pI 4.0 to 7.0
Spot 27α: Molecular weight 30 to 80 kDa, pI 4.0 to 7.0
Spot 28α: Molecular weight 20 to 60 kDa, pI 4.0 to 7.0
Spot 29α, 30α, 31α, 32α, 33α: Molecular weight 20 to 60 kDa, pI 4.0 to 8.0
Spot 34α: Molecular weight 20 to 60 kDa, pI 6.0 to 10.0
Spot 35α: Molecular weight 30 to 80 kDa, pI 6.0 to 10.0
Spot 36α, 37α: Molecular weight 20 to 60 kDa, pI 4.0 to 8.0
Spot 38α: Molecular weight 80 to 160 kDa, pI 6.0 to 10.0
Spot 39α: Molecular weight 10 to 50 kDa, pI 6.0 to 10.0
Spot 40α, 56α: Molecular weight 10 to 50 kDa, pI 6.0 to 10.0
Spot 41α: Molecular weight 10 to 50 kDa, pI 6.0 to 10.0
Spot 42α, 43α: Molecular weight 10 to 50 kDa, pI 6.0 to 10.0
Spot 44α: Molecular weight 10 to 50 kDa, pI 4.0 to 7.0
Spot 45α: Molecular weight 5 to 40 kDa, pI 4.0 to 7.0
Spot 46α: Molecular weight 5 to 40 kDa, pI 4.0 to 7.0
Spot 47α: Molecular weight 5 to 40 kDa, pI 4.0 to 7.0
Spot 48α: Molecular weight 5 to 40 kDa, pI 4.0 to 8.0
Spot 49α: Molecular weight 5 to 20 kDa, pI 4.0 to 7.0
Spot 50α: Molecular weight 5 to 40 kDa, pI 3.0 to 6.0
Spot 51α: Molecular weight 10 to 50 kDa, pI 3.0 to 6.0
Spot 52α: Molecular weight 20 to 60 kDa, pI 4.0 to 7.0
Spot 53α: Molecular weight 10 to 50 kDa, pI 4.0 to 8.0
Spot 54α: Molecular weight 10 to 50 kDa, pI 4.0 to 8.0
Spot 55α: Molecular weight 40 to 80 kDa, pI 6.0 to 10.0

### Example 3: Mass spectrometry and identification of antigens

The amino acid sequences of the antigens that form the 56 protein spots were identified by mass spectroscopy.

To be specific, protein extraction and mass spectroscopy were done by the following procedure.
(1) Soybeans were subjected to protein extraction, two-dimensional electrophoresis and transfer to membrane by following the procedures described in Example 1 and 2, and the resulting membrane was stained by shaking in a solution of 0.008% Direct blue in 40% ethanol and 10% acetic acid.
(2) Then, the membrane was decolorized by repeating a 5-minute treatment with 40% ethanol and 10% acetic acid three times, washed with water for 5 minutes, and then dried by air.
(3) A protein spot of interest was cut out with a clean cutter blade and put into a centrifugal tube. The cut membrane was subjected to hydrophilization with 50 µL of methanol, followed by washing with 100 µL of water twice and then centrifugal cleaning. Thereafter, 20 µL of 20 mM NH₄HCO₃ and 50% acetonitrile were added.
(4) 1 µL of 1 pmol/µL lysyl endopeptidase (produced by WAKO) was added, and the solution was left to stand at 37°C for 60 minutes and then collected in a new centrifugal tube. After 20 µL of 20 mM NH₄HCO₃ and 70% acetonitrile were added to the membrane, the membrane was immersed therein at room temperature for 10 minutes, and the resulting solution was further collected. The solution was dissolved with 0.1% formic acid and 10 µL of 4% acetonitrile and transferred to a tube.
(5) The collected solution was dried under reduced pressure, dissolved with 15 µL of solution A (a 0.1% formic acid/4% acetonitrile solution), and analyzed by mass spectroscopy (ESI-TOF5600, produced by AB Sciex).
(6) Identification of proteins based on the MS data obtained with the mass spectrometer was done by searching the NCBI database.

### Results

The mass spectrometry of the spots resulted in the detection of the following amino acid sequences. Furthermore, the spots were identified as the following proteins by the analysis of the mass data obtained from the mass spectrometer for the spots at NCBI.

### Spot 35α:

The amino acid sequences set forth in SEQ ID NOs: 461 to 466 were detected by the mass spectrometry. The protein identified from this mass data was elongation factor 1-alpha (NCBI accession number XP_003553292.1, amino acid sequence: SEQ ID NO: 460, encoding nucleotide sequence: SEQ ID NO: 459).

The amino acid sequences set forth in SEQ ID NOs: 469 to 471 were detected by the mass spectrometry. The protein identified from this mass data was elongation factor 1-alpha (NCBI accession number XP_003547695.1, amino acid sequence: SEQ ID NO: 468, encoding nucleotide sequence: SEQ ID NO: 467).

The amino acid sequences set forth in SEQ ID NOs: 474 to 477 were detected by the mass spectrometry. The protein identified from this mass data was elongation factor-1A isoform X1 (NCBI accession number XP_006600131.1, amino acid sequence: SEQ ID NO: 473, encoding nucleotide sequence: SEQ ID NO: 472).

### Spot 38α

The amino acid sequences set forth in SEQ ID NOs: 496 to 513 were detected by the mass spectrometry. The protein identified from this mass data was seed biotin-containing protein SBP65-like isoform X1 (NCBI accession number XP_003526237.1, amino acid sequence: SEQ ID NO: 495, encoding nucleotide sequence: SEQ ID NO: 494).

### Spot 48α:

The amino acid sequences set forth in SEQ ID NOs: 566 to 570 were detected by the mass spectrometry. The protein identified from this mass data was uncharacterized protein LOC100306570 (NCBI accession number NP_001236895.1, amino acid sequence: SEQ ID NO: 565, encoding nucleotide sequence: SEQ ID NO: 564).

### Example 4: Identification of epitopes (1)

Identification of epitopes was carried out by the following procedure as to each of the antigen proteins identified in Example 3.

### (A) Epitope mapping

Epitope mapping was carried out using a library of overlapping peptide fragments (length: 15 amino acids) corresponding to the sequences of proteins bound patient-specifically to IgE antibodies in serum, which were obtained as a result of 2D-western of soybeans.

The peptides to be synthesized were shifted by 10 amino acids. Thus, each peptide had an overlap of 5 amino acids with each of the peptides previous and subsequent thereto.

For preparation of peptide arrays, peptide synthesis of interest was carried out in incremental steps on a modified cellulose membrane by the Intavis SPOT synthesis technique, which is a technique of synthesizing peptides of interest through 9-fluorenylmethoxycarbonyl (Fmoc) chemical reaction, using an automated synthesis apparatus (Intavis MultiPep RS). The aforementioned modified cellulose membrane (hereinafter also referred to as the membrane) bound to the obtained peptides of interest was used in epitope mapping.

The presence or absence of binding, to each peptide fragment, of an IgE antibody present in the serum of a patient allergic to a soybean was measured by immunoblotting using the membrane on which the peptide fragments were synthesized by SPOT. Immunoblotting of the membrane was carried out according to the following procedure using, as a primary antibody, a serum sample from an allergic patient, and anti-human IgE-HRP as a secondary antibody.
(1) The membrane was subjected to hydrophilization with methanol for 5 minutes.
(2) The hydrophilized membrane was shaken overnight in a 5% skim milk/PBST solution (a PBS buffer containing 0.1% Tween 20 nonionic surfactant) at 4°C.
(3) The membrane was washed with a PBST solution three times.
(4) The serum sample (1:40, a 5% skim milk/PBST solution) was added thereto as a primary antibody and shaken overnight at 4°C. In this operation, a protease inhibitor (Complete, produced by Roche) was added.
(5) The membrane was washed with a PBST solution three times.
(6) Anti-human IgE-HRP (1:10,000, a 5% skim milk/PBST solution) was added thereto as a secondary antibody and shaken at room temperature for one hour.
(7) The membrane was washed with a PBST solution three times.
(8) The membrane was left to stand in ECL plus (produced by Thermo Fisher Scientific) for 5 minutes.
(9) The membrane treated as described above in (1) to (8) was subjected to fluorescent image scanning using Amersham Imager 600.

Peptide fragments bound to IgE antibodies from allergic patients in immunoblotting were used as candidate peptides of epitopes.

### (B) Overlapping

Thereafter, for the sequences of peptides bound patient-specifically to IgE antibodies in serum in the epitope mapping described above in (A), a library of overlapping peptide fragments (length: 10 amino acids) was obtained as sequences having the sequences of these peptides as well as additional sequences subsequent to the peptides in the amino acid sequences of antigen proteins on which the epitopes were detected. Synthesis of this library was carried out by the Fmoc chemical synthesis method in the same manner as in (A) mentioned above. The peptides to be synthesized were shifted by one amino acid. Thus, each peptide had an overlap of 9 amino acids with the peptide subsequent thereto.

The presence or absence of binding of an IgE antibody from an allergic patient to each peptide fragment in this library of overlapping peptide fragments was measured by immunoblotting in the same manner as in (A) mentioned above. A common sequence of peptides bound to IgE antibodies from allergic patients was identified to determine peptides having the minimum number of amino acids functioning as an epitope. The peptides determined in (B) are referred to as the "original sequences".

As one example of the results, the results of determining the original sequences by the overlapping technique as to a partial sequence (a region of amino acids 571-590 of SEQ ID NO: 495/SEQ ID NO: 964 of seed biotin-containing protein SBP65-like isoform X1 (spot 38α) are shown in Fig. 4. Strong binding of polypeptides having the amino acid sequences of SEQ ID NOs: 965-967 to the IgE antibody from the allergic patient was observed. Slightly weak binding of SEQ ID NO: 968 to the IgE antibody from the allergic patient was observed. Binding of SEQ ID NOs: 969-975 to the IgE antibody from the allergic patient was not observed. As a result, SEQ ID NO: 864, a common sequence of SEQ ID NOs: 965-968, was identified as an original sequence.

### (C) Alanine scanning

From the "original sequences" determined as described above in (B), a library of peptide fragments in which N-terminal amino acids were substituted one by one by alanine according to a technique called alanine scanning (Non Patent Literature 1) was prepared by the Fmoc chemical synthesis method in the same manner as in (A) mentioned above. The presence or absence of binding of an IgE antibody present in the serum of an allergic patient to each peptide fragment was measured by immunoblotting in the same manner as in (A) mentioned above. Amino acids at positions where the binding activity against IgE antibodies from allergic patients was lost by the substitution by alanine were confirmed as positions important for exertion of antigenicity. Also, Amino acids at positions where the binding activity against IgE antibodies from allergic patients was maintained even after the substitution by alanine were confirmed as substitutable positions that were not important for exertion of antigenicity. Common sequences (consensus sequences) important for exertion of antigenicity were found by this analysis.

As one example of the results, the results of performing alanine scanning as to the amino acid sequence of SEQ ID NO: 864 identified in (B) are shown in Fig. 5. Peptides having SEQ ID NOs: 977-979 in which the amino acids at positions 1-3, respectively, of SEQ ID NO: 864 were substituted by alanine lost their binding activity against IgE antibodies from allergic patients. Thus, the amino acids at positions 1-3 of SEQ ID NO: 864 were identified as amino acids important for the binding to IgE antibodies from allergic patients.

### (D) Results

### (35β) Epitope of elongation factor 1-alpha (spot 35α)

SEQ ID NOs: 849, 850, 853, and 856 were identified as epitopes of elongation factor 1-alpha by the procedure of (B) mentioned above.

Amino acids important for binding to IgE antibodies from allergic patients were identified by the procedure of (C) mentioned above as to each of SEQ ID NOs: 849, 850, 853 and 856.

It was revealed for SEQ ID NO: 849 that the amino acids at positions 5, 6, 10, 11 and 15 are amino acids particularly important for the binding to IgE antibodies from allergic patients, and the amino acids at positions 7 and 8 are amino acids that influence the binding to IgE antibodies from allergic patients. The amino acids at positions 1-4, 9 and 12-14 of SEQ ID NO: 849 had no influence on the binding activity against IgE antibodies from allergic patients when substituted by alanine.

It was revealed for SEQ ID NO: 853 that the amino acids at positions 1-3, 5 and 6 are amino acids particularly important for the binding to IgE antibodies from allergic patients, and the amino acid at position 4 is an amino acid that influences the binding to IgE antibodies from allergic patients. The amino acids at positions 7-9 of SEQ ID NO: 853 had no influence on the binding activity against IgE antibodies from allergic patients when substituted by alanine.

It was revealed for SEQ ID NO: 856 that the amino acids at positions 3, 5 and 6 are amino acids particularly important for the binding to IgE antibodies from allergic patients, and the amino acids at positions 1 and 2 are amino acids that influence the binding to IgE antibodies from allergic patients. The amino acids at positions 4 and 7-10 of SEQ ID NO: 856 had no influence on the binding activity against IgE antibodies from allergic patients when substituted by alanine.

For SEQ ID NO: 850, all the amino acids were amino acids particularly important for the binding to IgE antibodies from allergic patients.

### (38β) Epitope of seed biotin-containing protein SBP65-like isoform X1 (spot 38α)

SEQ ID NOs: 864, 867, 869, 871, 874, 877, 880, 882, 884, 887, 889, and 892 were identified as epitopes of seed biotin-containing protein SBP65-like isoform X1 by the procedure of (B) mentioned above.

Amino acids important for binding to IgE antibodies from allergic patients were identified by the procedure of (C) mentioned above as to each of SEQ ID NOs: 864, 867, 869, 871, 874, 877, 880, 882, 884, 887, 889, and 892.

It was revealed for SEQ ID NO: 864 that the amino acids at positions 1-3 are amino acids particularly important for the binding to IgE antibodies from allergic patients. The amino acids at positions 4-7 of SEQ ID NO: 864 had no influence on the binding activity against IgE antibodies from allergic patients when substituted by alanine.

It was revealed for SEQ ID NO: 867 that the amino acids at positions 2-4 are amino acids particularly important for the binding to IgE antibodies from allergic patients, and the amino acid at position 1 is an amino acid that influences the binding to IgE antibodies from allergic patients. The amino acids at positions 5-10 of SEQ ID NO: 867 had no influence on the binding activity against IgE antibodies from allergic patients when substituted by alanine.

It was revealed for SEQ ID NO: 869 that the amino acids at positions 1, 3 and 15 are amino acids particularly important for the binding to IgE antibodies from allergic patients. The amino acids at positions 2 and 4-14 of SEQ ID NO: 869 had no influence on the binding activity against IgE antibodies from allergic patients when substituted by alanine.

It was revealed for SEQ ID NO: 871 that the amino acids at positions 8, 9 and 15 are amino acids particularly important for the binding to IgE antibodies from allergic patients. The amino acids at positions 1-7 and 10-14 of SEQ ID NO: 871 had no influence on the binding activity against IgE antibodies from allergic patients when substituted by alanine.

It was revealed for SEQ ID NO: 874 that the amino acids at positions 4-8 and 10 are amino acids particularly important for the binding to IgE antibodies from allergic patients, and the amino acids at positions 1-3 are amino acids that influence the binding to IgE antibodies from allergic patients. The amino acid at position 9 of SEQ ID NO: 874 had no influence on the binding activity against IgE antibodies from allergic patients when substituted by alanine.

It was revealed for SEQ ID NO: 877 that the amino acids at positions 1-6, 9 and 10 are amino acids particularly important for the binding to IgE antibodies from allergic patients, and the amino acids at positions 7 and 8 are amino acids that influence the binding to IgE antibodies from allergic patients. The amino acids at positions 11-15 of SEQ ID NO: 877 had no influence on the binding activity against IgE antibodies from allergic patients when substituted by alanine.

It was revealed for SEQ ID NO: 880 that the amino acids at positions 2, 4-6, 8 and 9 are amino acids particularly important for the binding to IgE antibodies from allergic patients, and the amino acids at positions 7 and 12-15 are amino acids that influence the binding to IgE antibodies from allergic patients. The amino acids at positions 1, 3, 10 and 11 of SEQ ID NO: 880 had no influence on the binding activity against IgE antibodies from allergic patients when substituted by alanine.

It was revealed for SEQ ID NO: 882 that the amino acids at positions 1 and 5-9 are amino acids particularly important for the binding to IgE antibodies from allergic patients. The amino acids at positions 2-4 and 10 of SEQ ID NO: 882 had no influence on the binding activity against IgE antibodies from allergic patients when substituted by alanine.

It was revealed for SEQ ID NO: 884 that the amino acids at positions 5-10 are amino acids particularly important for the binding to IgE antibodies from allergic patients. The amino acids at positions 1-4 of SEQ ID NO: 884 had no influence on the binding activity against IgE antibodies from allergic patients when substituted by alanine.

It was revealed for SEQ ID NO: 887 that the amino acids at positions 2, 4, 5 and 14 are amino acids particularly important for the binding to IgE antibodies from allergic patients, and the amino acids at positions 6, 10, 12, 13 and 15 are amino acids that influence the binding to IgE antibodies from allergic patients. The amino acids at positions 1, 3, 7-9 and 11 of SEQ ID NO: 887 had no influence on the binding activity against IgE antibodies from allergic patients when substituted by alanine.

It was revealed for SEQ ID NO: 889 that the amino acids at positions 3-7 are amino acids particularly important for the binding to IgE antibodies from allergic patients. The amino acids at positions 1, 2 and 8-10 of SEQ ID NO: 889 had no influence on the binding activity against IgE antibodies from allergic patients when substituted by alanine.

It was revealed for SEQ ID NO: 892 that the amino acids at positions 5-10 are amino acids particularly important for the binding to IgE antibodies from allergic patients, and the amino acids at positions 3 and 4 are amino acids that influence the binding to IgE antibodies from allergic patients. The amino acids at positions 1 and 2 of SEQ ID NO: 892 had no influence on the binding activity against IgE antibodies from allergic patients when substituted by alanine.

### (48β) Epitope of uncharacterized protein LOC100306570 (spot 48α)

SEQ ID NOs: 921 and 922 were identified as epitopes of uncharacterized protein LOC100306570 by the procedure of (B) mentioned above.

Amino acids important for binding to IgE antibodies from allergic patients were identified by the procedure of (C) mentioned above as to each of SEQ ID NOs: 921 and 922.

It was revealed for SEQ ID NO: 921 that the amino acids at positions 7-9 are amino acids particularly important for the binding to IgE antibodies from allergic patients, and the amino acid at position 5 is an amino acid that influences the binding to IgE antibodies from allergic patients. The amino acids at positions 1-4 and 6 of SEQ ID NO: 921 had no influence on the binding activity against IgE antibodies from allergic patients when substituted by alanine.

For SEQ ID NO: 922, all the amino acids were amino acids particularly important for the binding to IgE antibodies from allergic patients.

### Example 6: Study on cross-reactivity

Amino acids important for binding activity against IgE antibodies from allergic patients were identified as to SEQ ID NO: 871, one of the polypeptides in spot 38β, to obtain SEQ ID NO: 870 (hereinafter also referred to as a key sequence). A protein having this key sequence was compared against NCBI protein data and analyzed by PHI-BLAST. As a result, peach-derived kinesin-like protein KIN-7C comprising SEQ ID NO: 984 was identified as an amino acid sequence corresponding to the key sequence, and apple-derived uncharacterized protein LOC103433727 isoform X2 comprising SEQ ID NO: 985 was identified as an amino acid sequence corresponding to the key sequence.

The binding activity of peptides comprising the amino acid sequences of SEQ ID NOs: 871, 984 and 985 against an IgE antibody from one patient having allergies to a soybean, a peach and an apple was confirmed by ELISA. The peptides were synthesized such that the peptides were N-terminally biotinylated by the Fmoc method.

To be specific, ELISA was carried out according to the following procedure.
(1) The concentrations of the biotinylated peptides were adjusted to 1 µg/mL with PBST.
(2) Each peptide solution was added at 40 µL to each well of a 384-well plate coated with streptavidin, and shaken at room temperature for one hour. After collection of the solution, the wells were washed with PBS five times.
(3) A 1:5 dilution of Blocking one (diluted with MQ) was added at 40 µL thereto and shaken at room temperature for 15 minutes. After removal of the solution, the wells were washed with PBS five times.
(4) A diluted serum solution (1:10, diluting solution: Blocking one produced by Nacalai Tesque, Inc.) was added at 40 µL thereto and shaken at room temperature for one hour. After removal of the solution, the wells were washed with PBS five times.
(5) A diluted secondary antibody solution (1:5000, diluting solution: Blocking one produced by Nacalai Tesque, Inc.) was added at 40 µL thereto and shaken at room temperature for one hour. After removal of the solution, the wells were washed with PBS five times.
(6) 1-Step Ultra TMB-ELISA (Thermo Fisher Scientific) was added at 40 µL thereto and shaken at room temperature for 15 minutes.
(7) 2 M H₂SO₄ was added at 40 µL thereto. Absorbance at 450 nm was measured.

The results are shown in Fig. 6. As shown in Fig. 6, the peptides comprising the amino acid sequences of SEQ ID NOs: 871, 984 and 985 exhibited binding activity against the IgE antibody from the patient having allergies to a soybean, a peach and an apple. Also, these peptides bound more strongly to the IgE antibody from the allergic patient than to an IgE antibody from a healthy subject who was not an allergic patient. This indicates that this key sequence can be used for detecting the cross-reactivity of an allergen.

This result also indicates that the polypeptides (1β) to (60β) of the present invention can also be used for the detection of the cross-reactivity of an allergen.

## Claims

1. A polypeptide specifically binding to an IgE antibody from an allergic patient, the polypeptide being (35β) or (48β) of the following:
(35β) a polypeptide comprising at least one amino acid sequence selected from the group consisting of SEQ ID NOs: 847-856; and
(48β) a polypeptide comprising at least one amino acid sequence selected from the group consisting of SEQ ID NOs: 919-922.

2. The polypeptide according to claim 1, wherein the number of amino acid residues is 500 or less.

3. A kit for diagnosing an allergy, comprising at least one of polypeptides according to any claim 1 or 2.

4. A composition for diagnosing an allergy, the composition comprising at least one of polypeptides according to claim 1 or 2 as an antigen.

5. A method for providing an indicator for diagnosing an allergy in a subject, the method comprising the steps of:
(i) contacting a sample obtained from the subject with an antigen, wherein the sample is a solution comprising an Ig antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is at least one of polypeptides according to claim 1 or 2.

6. A pharmaceutical composition comprising at least one of polypeptides according to claim 1 or 2.

7. The pharmaceutical composition according to claim 6, wherein the pharmaceutical composition is intended for the treatment of an allergy.

8. A tester for determining the presence or absence of an antigen in an object of interest, the tester comprising an antibody that binds to at least one of polypeptides according to claim 1 or 2.

9. A food raw material or an edible processed product in which an antigen is eliminated or reduced, or a polypeptide is cleaved or removed, wherein the antigen is at least one of polypeptides according to claim 1 or 2.

10. A method for producing an edible processed product in which an antigen is eliminated or reduced, or a polypeptide is cleaved or removed, the method comprising the step of confirming that the antigen is eliminated or reduced, or the polypeptide is cleaved or removed, in a production process of the processed product, wherein the antigen is at least one of polypeptides according to claim 1 or 2.
